(19) Europäisches Patentamt
European Patent Office
Office européen des brevets

(11) Veröffentlichungsnummer: **0 666 269 A2**

(12) **EUROPÄISCHE PATENTANMELDUNG**

(21) Anmeldenummer: **95106043.3**

(22) Anmeldetag: **23.04.90**

(51) Int. Cl.6: **C07H 19/04**, A61K 31/70, C12Q 1/68, C07H 21/00

Diese Anmeldung ist am 22 - 04 - 1995 als Teilanmeldung zu der unter INID-Kode 60 erwähnten Anmeldung eingereicht worden.

(30) Priorität: **24.07.89 DE 3924424**

(43) Veröffentlichungstag der Anmeldung: **09.08.95 Patentblatt 95/32**

(60) Veröffentlichungsnummer der früheren Anmeldung nach Art. 76 EPÜ: **0 484 333**

(84) Benannte Vertragsstaaten: **AT BE CH DE DK ES FR GB GR IT LI LU NL SE**

(71) Anmelder: **BOEHRINGER MANNHEIM GMBH**
**D-68298 Mannheim (DE)**

(72) Erfinder: **Seela, Frank, Prof. Dr.**
**Gutenbergerstrasse 44**
**D-49076 Osnabrück (DE)**
Erfinder: **Bourgeois, Werner**
**Wilkienskamp 16**
**D-49090 Osnabrück (DE)**
Erfinder: **Gumbiowski, Rainer**
**Rappstrasse 1**
**D-49084 Osnabrück (DE)**
Erfinder: **Roeling, Angelika**
**Hansaring 44**
**D-48155 Münster (DE)**
Erfinder: **Rosemeyer, Helmut, Dr.**
**Luermannstrasse 39**
**D-49076 Osnabrück (DE)**
Erfinder: **Mertens, Alfred, Dr.**
**Beethovenstrasse 20**
**D-69198 Mannheim (DE)**
Erfinder: **Zilch, Harald, Dr.**
**Asenweg 24**
**D-68305 Mannheim (DE)**
Erfinder: **König, Bernhard, Dr.**
**Duerrbergstrasse 28**
**D-82335 Berg (DE)**
Erfinder: **Koch, Edith, Dr.**
**Lagoner Strasse 18**
**D-82377 Penzberg (DE)**

(54) **Nucleosid-Derivate und deren Verwendung als Arzneimittel.**

(57) Die Erfindung betrifft Nucleosid- und Nucleotid-Derivate, Verfahren zur Herstellung dieser Verbindungen, die Verwendung dieser Nucleosid-Derivate als Arzneimittel sowie deren Verwendung bei der Sequenzierung von Nucleinsäuren.

Gegenstand der vorliegenden Erfindung sind neue Nucleosid- und Nucleotid-Derivate der allgemeinen Formel I

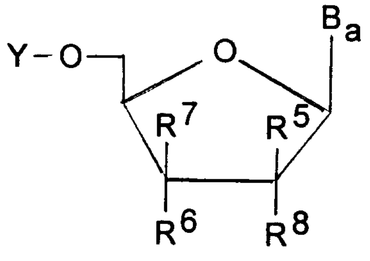

(I)

in der

EP 0 666 269 A2

| | |
|---|---|
| $B_a$ | eine gegebenenfalls substituierte Benzimidazolyl-, Pyrrolopyridinyl-, Imidazopyridinyl-, Triazolopyrimidinyl, Imidazotriazinyl- oder Imidazopyrimidinylgruppe bedeutet, |
| $R^5$, $R^6$, $R^7$ und $R^8$ | jeweils Wasserstoff bzw. einer oder zwei der Reste $R^5$, $R^6$, $R^7$ oder $R^8$ eine Hydroxy-, Halogen-, Cyano- oder Azidogruppe bedeuten, oder $R^5$ und $R^7$ zusammen eine weitere Bindung zwischen C-2' und C-3' darstellen können, und |
| Y | Wasserstoff oder eine $C_1$-$C_7$-Alkylcarbonyl, Monophosphat-, Diphosphat-oder Triphosphatgruppe darstellt, |

sowie deren mögliche $\alpha$- oder $\beta$-Anomere, $N^7$-, $N^8$- oder $N^9$-Regioisomere (Purin-Nomenklatur), Tautomere und Salze, sowie Nucleinsäuren, die Verbindungen der Formel I als Bausteine enthalten.

Die Erfindung betrifft Nucleosid- und Nucleotid-Derivate, Verfahren zur Herstellung dieser Verbindungen, die Verwendung dieser Nucleosid-Derivate als Arzneimittel sowie deren Verwendung bei der Sequenzierung von Nucleinsäuren.

Gegenstand der vorliegenden Erfindung sind neue Nucleosid- und Nucleotid-Derivate der allgemeinen Formel I

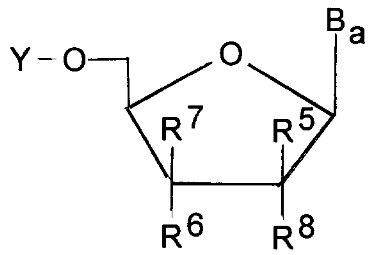

(I)

in der

$B_a$ eine Benzimidazolyl- (B), Pyrrolopyridinyl- (c), Imidazopyridinyl- (D), Triazolopyrimidinyl- (E), Imidazotriazinyl- (F) oder Imidazopyrimidinylgruppe (G) bedeutet,

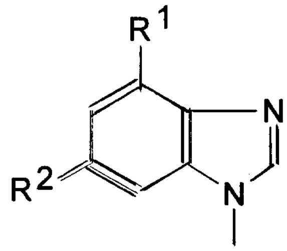

(B)

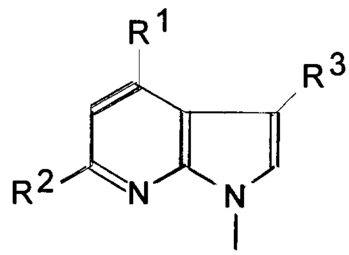

(C)

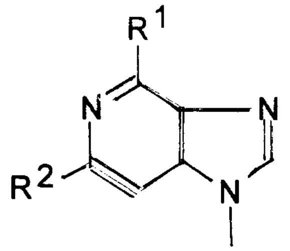

(D)

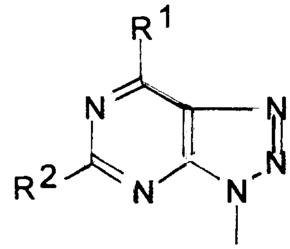

(E)

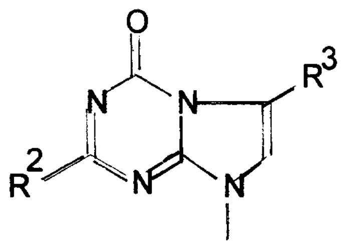

(F)

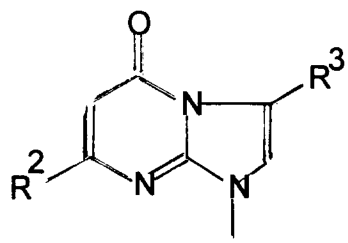

(G)

wobei

$R^1$, $R^2$, $R^3$, die gleich oder verschieden sein können, Wasserstoff, Halogen, eine $C_1$-$C_7$-Alkyl-, $C_2$-$C_7$-Alkenyl-, Hydroxy-, Mercapto-, $C_1$-$C_7$-Alkylthio-, $C_1$-$C_7$-Alkyloxy-, $C_2$-$C_7$-Alkenyloxy-, Ar-$C_1$-$C_5$-alkyl-, Ar-$C_2$-$C_5$-alkenyl-, Ar-$C_1$-$C_5$-alkyloxy-, Ar-$C_2$-$C_5$-alkenyloxy-, Aryloxy-, Nitro-, Amino-$C_1$-$C_7$-alkyl-, $C_1$-$C_7$-Alkylamino-$C_1$-$C_7$-alkyl-, Di-$C_1$-$C_7$-alkylamino-$C_1$-$C_7$-alkyl-, Amino-, eine substituierte Aminogruppe-$NHR^4$, bzw.-$N(R^4)_2$, oder eine Iminogruppe -N = CH-$R^4$ bedeuten,
wobei $R^4$ eine $C_1$-$C_7$-Alkyl-, $C_2$-$C_7$-Alkenyl-, $C_3$-$C_7$-Cycloalkyl-, $C_3$-$C_7$-Cycloalkyl-$C_1$-$C_7$-alkyl-, $C_3$-$C_7$-Cycloalkenyl-, $C_1$-$C_7$-Alkoxy-$C_1$-$C_7$-alkyl-, Halogen-$C_1$-$C_7$-alkyl-, Hydroxy-$C_1$-$C_7$-alkyl-, Ar-$C_1$-$C_5$-alkyl-, Ar-$C_2$-$C_5$-alkenyl-, Hetaryl-$C_1$-$C_5$-alkyl- oder Hetaryl-$C_2$-$C_5$-alkenylgruppe, wobei der Aryl- bzw. Hetarylteil ein-, zwei- oder dreifach durch $C_1$-$C_6$-Alkyl, $C_2$-$C_6$-Alkenyl-, $C_1$-$C_6$-Alkoxy, Halogen oder Hydroxy substitiert sein kann, oder $R^4$ eine Amino-$C_1$-$C_7$-alkyl-, $C_1$-$C_7$-Alkylamino-$C_1$-$C_7$-alkyl- oder Di-$C_1$-$C_7$-alkylamino-$C_1$-$C_7$-alkylgruppe bedeuten kann und im Fall der -$NHR^4$ und -N = CH-$R^4$ Gruppe, $R^4$ zusätzlich eine Amino-, $C_1$-$C_7$-Alkylamino-, Di-$C_1$-$C_7$-alkylamino- oder $C_1$-$C_7$-Alkoxygruppe sein kann, oder im Fall der $N(R^4)_2$-Gruppe die beiden Stickstoffsubstituenten $R^4$ zusammen eine $C_1$-$C_7$-Alkylidengruppe bilden, die ihrerseits durch eine $C_1$-$C_7$-Alkoxy-, $C_1$-$C_7$-Alkylamino- oder Di-$C_1$-$C_7$-Alkylaminogruppe substituiert sein kann,
wobei unter "Ar" die Phenyl- und Naphthylgruppe verstanden wird, "Hetaryl"-gruppen sind vorzugsweise die Furanyl-, Thienyl- oder Pyridylgruppe,

$R^5$, $R^6$, $R^7$ und $R^8$ jeweils Wasserstoff bzw. einer oder zwei der Reste $R^5$, $R^6$, $R^7$ oder $R^8$ eine Hydroxy-, Halogen-, Cyano- oder Azidogruppe bedeuten, oder $R^5$ und $R^7$ zusammen eine weitere Bindung zwischen C-2' und C-3' darstellen können, und

Y Wasserstoff oder eine $C_1$-$C_7$-Alkylcarbonyl-, Monophosphat-, Diphosphat-oder Triphosphatgruppe darstellt,

mit der Maßgabe, daß

a) für den Fall, daß $R^6$ eine Hydroxygruppe ist, $R^8$ nicht ein Wasserstoffatom oder eine Hydroxygruppe sein kann,

b) für den Fall, daß $B_a$ die Gruppe (D) und $R^2$ Wasserstoff ist, $R^1$ keine Chlor- oder Aminogruppe und $R^6$ kein Wasserstoffatom sein kann, und

c) für den Fall, daß $B_a$ die Gruppe (E) und $R^1$ die Aminogruppe ist, $R^5$ und $R^7$ gemeinsam keine Bindung darstellen können,

sowie deren mögliche $\alpha$- oder $\beta$-Anomere, $N^7$-, $N^8$- oder $N^9$-Regioisomere (Purin-Nomenklatur), Tautomere und Salze, sowie Nucleinsäuren, die Verbindungen der Formel I als Bausteine enthalten.

Ähnliche Verbindungen der Formel I sind aus EP-A-286,028 bekannt. Die vorliegenden erfindungsgemäßen Verbindungen unterscheiden sich strukturell von den bekannten Verbindungen durch die in der Definition von $B_a$ angegebenen Basen. Weiterhin weisen sie überraschende und überlegene Eigenschaften bzgl. der Verwendung als Arzneimittel auf.

Die Verbindungen der allgemeinen Formel I sind neue Verbindungen.

Aus dem Stand der Technik sind bereits eine Vielzahl von Ribofuranosyl-Derivaten ($R^6 = R^8 = OH$) sowie die entsprechenden Desoxy-ribofuranosyl-Derivate ($R^6 = OH$; $R^8 = H$) bekannt, die jedoch durch den Disclaimer a) nicht von der vorliegenden Erfindung umfaßt werden.

Aus der Literatur bekannt (Bioorg. Khim. 13, 1375, 1987) sind im Basenteil unsubstituierte Benzimidazole (Basentyp B), die im Zuckerteil in 3'-Stellung Halogen-, Azido- und Aminoreste enthalten und als Triphosphate hinsichtlich ihrer Substratspezifität für die DNA-Biosynthese in vitro untersucht wurden. Die Synthese der entsprechenden Nukleoside wurde ebenfalls publiziert (Z. Chem. 25, 180, 1985 und Synthesis 410, 1985). Diese Verbindungen werden jedoch vom vorliegenden Stoffanspruch nicht umfaßt.

Von den 3-Deazapurin-Nukleosiden (Purin-Nomenklatur; Basentyp D) ist die Synthese von Derivaten bekannt, die in 6-Stellung Chlor oder eine Aminogruppe enthalten und in der Ribose in 3'-Stellung durch Wasserstoff oder ein Chloratom substituiert sind (Nucleic Acids Res., 15, 1121, 1987 und Nucleosides Nucleotides 3, 413, 1984). Diese Verbindungen werden durch den Disclaimer b) vom Stoffanspruch nicht umfaßt. Eine pharmakologische Wirkung dieser Verbindungen ist nicht beschrieben.

In US-A-3,817,982 ist ein 8-Aza-6-amino-purin-Derivat (Basentyp E) mit einem 2',3'-Didehydro-2',3'-didesoxyriboserest beschrieben, das Verwendung als Antibiotikum, Virustatikum und bei DNA-Replikations-Studien finden kann. Diese Verbindung wird durch den Disclaimer c) vom Stoff- und Arzneimittelanspruch nicht umfaßt.

Ferner sind bereits einige Verbindungen der Formel I literaturbekannt, in denen Y ein Wasserstoffatom (Nucleoside) oder eine Alkylcarbonylgruppe bedeutet, und entweder $R^6$ und $R^8$ gleichzeitig Hydroxy (Ribose-Derivate), oder $R^6$ Hydroxy und $R^8$ ein Wasserstoffatom (2',3'-Didesoxyribose-Derivate) darstellen. Diese Verbindungen sind durch den Disclaimer a) vom Stoffanspruch ausgenommen. Das gleiche trifft zu für die aus EP-A-0,038,569 bekannten 2'-Desoxyribofuranosylnucleoside mit dem Basentyp (D), die eine entzündungshemmende Wirkung besitzen.

Außerdem sind aus EP-A-0,043,722 $\beta$-D-Arabinofüranosyl-nucleoside mit dem Basentyp (D) als antivirale Mittel bekannt. Bei diesen Verbindungen handelt es sich um Furanosyl-Derivate, in denen $R^5$ und $R^6$ jeweils eine Hydroxygruppe und R8 ein Wasserstoffatom bedeutet. Aufgrund des Disclaimers a) werden diese Derivate von den Ansprüchen nicht umfaßt.

Für den Fall, daß $B_a$ den Basentyp (B) oder (D) darstellt, so sind auch Gegenstand der vorliegenden Erfindung die $N^7$- und $N^9$-Regioisomere, und für den Fall, daß $B_a$ eine Triazolopyrimidingruppe (Basentyp E) ist, auch die entsprechenden $N^7$-, $N^8$- oder $N^9$-Regioisomere. Die Trennung der verschiedenen Regioisomere erfolgt nach an sich bekannten Methoden, wie zum Beispiel durch Säulenchromatographie.

Die "Alkyl-" oder "Alkenyl"-teile in der Definition der Substituenten $R^1$, $R^2$, $R^3$ und $R^4$ können geradkettig oder verzweigt sein und 1 - 7, vorzugsweise 1 - 4 Kohlenstoffatome enthalten. Ganz besonders bevorzugt sind die Methyl- und die Ethylgruppe, für $R^4$ außerdem noch die Propyl- und Isobutylgruppe.

Unter Halogen in der Definition der Substituenten $R^1$, $R^2$, und $R^3$ werden Fluor, Chlor, Brom und Jod verstanden, besonders bevorzugt ist das Fluor- und Chloratom.

Die in den Definitionen der Substituenten $R^1$, $R^2$, $R^3$ oder $R^4$ vorkommenden Aralkyl-, Hetarylalkyl- bzw. Aralkoxy-Reste enthalten vorzugsweise eine Alkylenkette mit 1-5, bzw. 1-3 Kohlenstoffatomen, die ein- oder zweifach mit einem aromatischen Rest, beispielsweise Phenyl- oder Naphthylrest, substituiert ist. Die Arylteile der zuvor genannten Aralkyl-, Aralkoxy- oder Hetarylalkylgruppen können ihrerseits ein-, zwei- oder dreifach durch eine Alkyl-, Hydroxy-, Halogen- oder Alkoxygruppe mit jeweils 1-6, bevorzugt 1-3 Kohlenstoffatomen substituiert sein. Als Aralkylgruppe ist besonders bevorzugt die Benzyl- und Hetarylmethylgruppe.

Als Aryloxy-Reste in der Definition der Substituenten $R^1$, $R^2$ und $R^3$ sind besonders Phenyloxy-Reste bevorzugt, die gegebenenfalls ein-, zwei- oder dreifach durch weitere Substituenten, wie beispielsweise Nitro-, Alkyl- und Alkoxygruppen substituiert sein können, wobei die Alkyl- und Alkoxygruppen 1-6 Kohlenstoffatome enthalten können.

Die in der Definition der Substituenten $R^1$, $R^2$, und $R^3$ vorkommende Aminogruppe, die gegebenenfalls ein- oder zweifach durch $R^4$ substituiert sein kann, enthält als mögliche Substituenten vorzugsweise Alkyl-, Alkenyl-, Cycloalkyl-, Alkoxyalkyl-, Halogenalkyl-, Aralkyl- und Di-alkylaminoalkylgruppen, wobei die Alkyl- und Alkenylteile der vorgenannten Gruppen vorzugsweise 1-5, bzw. 1-3 Kohlenstoffatome enthalten.

Die beiden Stickstoffsubstituenten $R^4$ können auch zusammen einen Alkyliden, vorzugsweise einen Methyliden-Rest darstellen, der seinerseits durch Alkoxy oder durch substituierte Aminogruppen substituiert sein kann. Ein ganz bevorzugter Substituent dieser Art ist die Dimethylaminomethyliden-Gruppe.

Die Monophosphatgruppe ist die Gruppe $-PO(OH)_2$, die Diphosphatgruppe, die Gruppe $-P_2O_3(OH)_3$ und die Triphosphatgruppe bedeutet die Gruppe $-P_3O_5(OH)_4$.

Als mögliche Salze kommen vor allem Alkali-, Erdalkali-und Ammoniumsalze der Phosphatgruppen in Frage. Als Alkalisalze sind Lithium-, Natrium- und Kaliumsalze bevorzugt. Als Erdalkalisalze kommen insbesondere Magnesium- und Calciumsalze in Frage. Unter Ammoniumsalzen werden erfindungsgemäß Salze verstanden, die das Ammoniumion enthalten, das bis zu vierfach durch Alkylreste mit 1 - 4 Kohlenstoffatomen oder/und Aralkylreste, bevorzugt Benzylreste, substituiert sein kann. Die Substituenten können hierbei gleich oder verschieden sein. Die Salze der Phosphate können auf bekannte Weise in die freien Säuren überführt werden.

Die Verbindungen der Formel I können basische Gruppen, insbesondere Amino-Gruppen enthalten, die mit geeigneten Säuren in Säureadditionsalze übergeführt werden können. Als Säuren kommen hierfür beispielsweise in Betracht: Salzsäure, Bromwasserstoffsäure, Schwefelsäure, Phosphorsäure, Fumarsäure, Bernsteinsäure, Weinsäure, Zitronensäure, Milchsäure, Maleinsäure oder Methansulfonsäure.

Besonders bevorzugte Verbindungen der allgemeinen Formel I sind solche, die als Glyceropentofuranoside bezeichnet werden, insbesondere deren Didesoxy- und Didesoxy-didehydro-Derivate. In diesem Sinne kommen die folgenden Bedeutungen von $R^5$-$R^8$ in Frage: $R^5$ und $R^7$ stellen vorzugsweise ein Wasserstoffatom dar, bzw. $R^5$ und $R^7$ bilden gemeinsam eine Bindung (2',3'-Didesoxy-2',3'-didehydroribofüranosyl-Derivate).

Für $R^1$-$R^3$ kommen insbesondere die folgenden Gruppen in Frage: für $R^1$ Wasserstoff, Amino, Halogen, Nitro, $C_1$-$C_6$-Alkoxy, insbesondere Methoxy, für $R^2$ Wasserstoff, Amino oder Halogen, für $R^3$ Wasserstoff.

In Abhängigkeit vom Basentyp (B) - (G) kommen für die Reste $R^1$-$R^8$ vor allem die folgenden Gruppen bevorzugt in Frage:

Wenn $B_a$ die Gruppe (B) ist, dann bedeuten $R^1$-$R^3$ und $R^5$-$R^8$ vorzugsweise ein Wasserstoffatom.

Wenn $B_a$ die Gruppe (C) ist, dann bedeutet $R^1$ vorzugsweise ein Wasserstoffatom oder eine Amino- oder Nitrogruppe, und $R^2$, $R^3$, $R^5$-$R^8$ jeweils ein Wasserstoffatom, bzw. $R^5$ und $R^7$ zusammen auch eine Bindung.

Wenn $B_a$ die Gruppe (D) ist, dann bedeutet $R^1$ vorzugsweise eine Amino- oder Chlorgruppe und $R^2$, $R^3$, $R^5$-$R^8$ jeweils ein Wasserstoffatom.

Wenn $B_a$ die Gruppe (E) ist, dann bedeutet $R^1$ vorzugsweise eine Amino- oder $C_1$-$C_7$-Alkoxygruppe und $R^2$, $R^5$-$R^8$ jeweils ein Wasserstoffatom.

Wenn $B_a$ die Gruppe (F) ist, dann bedeutet $R^2$ vorzugsweise ein Wasserstoffatom oder eine Aminogruppe und $R^3$, $R^5$-$R^8$ vorzugsweise ein Wasserstoffatom.

Wenn $B_a$ die Gruppe (G) ist, dann bedeutet $R^2$ vorzugsweise ein Wasserstoffatom oder eine Amino- oder Chlorgruppe und $R^3$, $R^5$-$R^8$ ein Wasserstoffatom.

Die erfindungsgemäßen Verbindungen können in Analogie zu bekannten, verwandten Verbindungen hergestellt werden. Als besonders zweckmäßig hat sich zur Herstellung der Verbindungen der Formel I ein Verfahren erwiesen, bei dem man eine Verbindung der Formel II

$B_a$-X (II)

in der $B_a$ die oben angegebene Bedeutung hat, und X Wasserstoff oder eine Alkalimetallgruppe, wie z.B. Lithium oder Natrium bedeutet,
mit einer Verbindung der Formel III

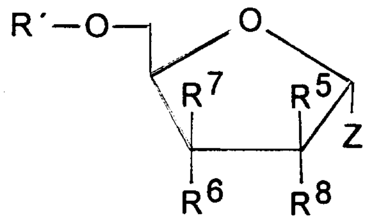

(III)

in der

$R^5$, $R^6$, $R^7$ und $R^8$ die oben angegebene Bedeutung haben, R' eine Sauerstoffschutzgruppe und Z eine reaktive Gruppe bedeutet,

zu einer Verbindung der Formel IV

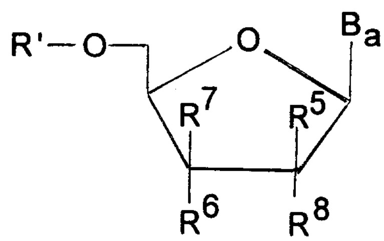

(IV)

in der Ba, $R^1$, $R^2$, $R^3$, $R^5$, $R^6$, $R^7$, $R^8$ und R' die oben angegebene Bedeutung haben, umsetzt und gegebenenfalls vorhandene Sauerstoffschutzgruppen abspaltet, und gewünschtenfalls anschließend Verbindungen der Formel I, in denen Y Wasserstoff bedeutet, in bekannter Weise in die Mono-, Di- oder Triphosphate überführt, und gewünschtenfalls erhaltene freie Basen bzw. Säuren in die entsprechenden Salze oder erhaltene Salze in die entsprechenden freien Basen bzw. Säuren umwandelt.

Die Verbindungen der Formel II werden mit den Verbindungen der Formel III umgesetzt, besonders vorteilhaft unter Phasentransferbedingungen. Unter den Bedingungen der Phasentransferkatalyse werden die Basen der Formel II in ein entsprechendes Anion überführt, beispielsweise durch 50%ige wässrige Natronlauge. Das so entstandene Anion wird durch einen Phasentransferkatalysator, beispielsweise Tris[2-(2-methoxyethoxy)ethyl]-amin, hydrophobiert und in die organische Phase transportiert, in der es mit der reaktiven Verbindung der Formel III abreagiert.

Als reaktive Gruppen Z in den Verbindungen der allgemeinen Formel III kommen vorzugsweise Halogen, Acetoxy und Alkoxygruppen in Frage. Die Hydroxygruppen des Zuckerrestes werden bei dieser Umsetzung in üblicher Weise durch dem Fachmann geläufige Sauerstoffschutzgruppen, beispielsweise Toluoyl-, Benzoyl-, Tetrahydropyranyl- oder Acetylgruppen, geschützt. Die Sauerstoffschutzgruppen können nach beendeter Umsetzung in bekannter Weise unter alkalischen Bedingungen wieder abgespalten werden, zweckmäßigerweise verwendet man eine 1M methanolische Methanolatlösung.

Es kann zweckmäßig sein, auch die Reste $R^1$, $R^2$ und $R^3$ während der Reaktion durch geeignete Schutzgruppen geschützt zu halten.

Eine weitere vorteilhafte Methode zur Herstellung der Verbindungen der Formel IV stellt das Fest-Flüssig-Phasentransfer-Verfahren unter Verwendung von festem, pulverförmigem Kaliumhydroxid, dem oben genannten Kryptanden, sowie den Verbindungen der Formeln II und III in einem aprotischen Lösungsmittel dar.

Die so geschützte Verbindung der Formel I wird mit einem Halogenid, zweckmäßigerweise einem Alkalihalogenid oder einem organischen Halogenid, bzw. mit einem Azid, zweckmäßigerweise mit einem Alkaliazid, wie z.B. Lithiumazid in allgemein bekannter Weise umgesetzt. Die OH-Gruppe am C-2'- oder C-3'-Atom wird dabei nucleophil durch das Halogenid bzw. Azid substituiert.

Verbindungen der Formel I, in denen $R^5$, $R^6$, $R^7$ oder $R^8$ eine Hydroxygruppe bedeuten, können nach vorherigem Schutz der 5'-Hydroxygruppe nach bekannten Methoden desoxygeniert werden, wobei Verbindungen der Formel I entstehen, in denen $R^5$, $R^6$, $R^7$ oder $R^8$ Wasserstoffbedeuten. Zum selektiven Schutz der Hydroxygruppe in 5'-Stellung stehen dem Fachmann bekannte Verfahren zur Verfügung. Beispielsweise hat sich in der Nucleotidchemie die 4,4'-Dimethoxy-triphenylmethylgruppe bewährt. Diese kann nach erfolgter Umsetzung wieder leicht durch milde Säurehydrolyse abgespalten werden, während die ebenfalls

säurelabile glycosidische Bindung unter diesen Bedingungen nicht hydrolysiert wird. Die Umsetzung des zu schützenden Nucleosids mit dem Sauerstoffschutzgruppenreagenz für die 5'-Hydroxygruppe wird in einem geeigneten organischen Lösungsmittel, zweckmäßigerweise getrocknetem Pyridin, mit einem leichten Überschuß des Sauerstoffschutzgruppenreagenzes sowie gegebenenfalls einer geeigneten Hilfsbase, beispielsweise N-Ethyldiisopropylamin, durchgeführt. Zur Desoxygenierung wird die Verbindung der allgemeinen Formel I, in der $R^5$, $R^6$, $R^7$ oder $R^8$ eine Hydroxygruppe vorstellt und bei der die 5'-Hydroxygruppe in vorstehender Weise geschützt worden ist und auch sonstige funktionelle Reste Schutzgruppen tragen, zunächst in ein 2'- oder 3'-O-Thiocarbonylderivat überführt, welches anschließend mit Tributylzinnhydrid radikalisch reduziert wird. Solche Methoden zur Desoxygenierung von 2'-Desoxynucleosiden zu 2'- und 3'-Didesoxynucleosiden sind dem Fachmann bekannt. Als besonders günstig hat sich die Methode der Barton-Desoxygenierung erwiesen (J. Chem. Soc., Perkin Trans. I (1975) 1574).

Verbindungen der Formel I, in denen $R^5$ und $R^7$ eine weitere Bindung zwischen C-2' und C-3' darstellen, können in Analogie zu bekannten verwandten Verbindungen (Robins, Hansske Tetrahedron Letters, 25, 367, 1984 und hierin zitierte Literatur) hergestellt werden. Als besonders zweckmäßig hat sich zur Herstellung dieser Verbindungen ein Verfahren erwiesen, bei dem man die entsprechenden Ribosen mit Acetoxyisobutyrylbromid umsetzt und die entstandenen Isomeren anschließend mit einem Reduktionsmittel wie z.B. dem Zink/Kupfer Paar oder ähnlichen Reduktionsmitteln reduziert und aus dem erhaltenen Rohprodukt nach Abspaltung der Schutzgruppe unter alkalischen Bedingungen die 2',3'-Didesoxy-2',3'-didehydro- Derivate erhält.

Neben diesem Verfahren sind in der Literatur weitere Verfahren zur Didesoxygenierung und gleichzeitigen Einführung der Doppelbindung beschrieben (vgl. Jain et al. J.Org.Chem. 39, 30, 1974; Robins et al. JACS 98, 8204 und 8213, 1976; Adachi et al. J.Org.Chem. 44, 1404, 1979; Mengel et al. Tetrah. Lett. 4203, 1977; Classon et al. Acta Chem. Scand. B 36, 251, 1982; Chu et al. J.Org.Chem. 54, 2217, 1989). Weiterhin können diese Verbindungen aus entsprechenden 2'-Desoxyribosen nach bekannten Verfahren (vgl. Horwitz et al. JACS 86, 1896, 1964; McCarthy et al. JACS 88, 1549, 1966; Samukov et al. Biorg. Khim. 9, 52, 1982) der Monodesoxygenierung unter gleichzeitiger Einführung der Doppelbindung hergestellt werden. Ein weiterer Weg zur Herstellung dieser Verbindungen ist die Umsetzung einer 2',3'-Didesoxy-2',3'didehydroribose mit einem entsprechend substituierten Basenderivat $B_a$ wie sie einem Fachmann aus der Literatur (vgl. z.B. EP-A-0,286,028) bekannt ist.

Die Phosphatgruppen werden in Verbindungen der allgemeinen Formel I, in denen Y Wasserstoff bedeutet, in bekannter Weise eingeführt. Die Monophosphate erhält man beispielsweise, indem man Verbindungen der Formel I, in denen Y Wasserstoff bedeutet, mit Phosphoroxychlorid in Trimethylphosphat phosphoryliert. Die auf diesem Wege erhaltenen Triethylammoniumsalze können in bekannter Weise in andere Salze durch Umsalzen überführt werden. Die Di- bzw. Triphosphate werden nach bekannten Methoden, vorzugsweise aus den Monophosphaten durch Umsetzung mit o-Phosphaten bzw. Pyrophosphaten erhalten. Ihre verschiedenen Salze können ebenfalls nach bekannten Methoden hergestellt werden.

Die Verbindungen der Formel II sind bekannte Verbindungen oder können in Analogie zu bekannten Verbindungen hergestellt werden. Derartige Herstellungsverfahren sind beispielsweise beschrieben in Chem. Ber. 110 (1977) 1462, J. Chem. Soc. 1960, 131 bzw. Tetrahedron Lett. 21 (1980) 3135.

Auch die Verbindungen der Formel III sind zum Teil bekannte Verbindungen. Bisher nicht beschriebene Verbindungen können in völliger Analogie zu den bekannten Verbindungen hergestellt werden. Die Herstellung einer solchen Verbindung ist beispielsweise beschrieben in Chem. Ber. 93 (1960) 2777 bzw. Synthesis 1984, 961.

Die Verbindungen der vorliegenden Erfindung weisen wertvolle pharmakologische Eigenschaften auf. Insbesondere eignen sie sich zur Therapie und Prophylaxe von Infektionen, die durch DNA-Viren wie z.B. das Herpes-Simplex-Virus, das Zytomegalie-Virus, Papova-Viren, das Varicella-Zoster-Virus oder Epstein-Barr-Virus oder RNA-Viren wie Toga-Viren oder insbesondere Retroviren wie die Onko-Viren HTLV-I und II, sowie die Lentiviren Visna und Humanes-Immunschwäche-Virus HIV-1 oder -2, verursacht werden.

Besonders geeignet erscheinen die Verbindungen der Formel I zur Behandlung der klinischen Manifestationen der retro-viralen HIV-Infektion beim Menschen, wie der anhaltenden, generalisierten Lymphadenopathie (PGL), dem fortgeschrittenen Stadium des AIDS-verwandten Komplex (ARC) und dem klinischen Vollbild von AIDS.

Überraschenderweise wurde nun gefunden, daß Verbindungen der allgemeinen Formel I die Vermehrung von DNA- bzw. RNA-Viren auf der Stufe der virusspezifischen DNA- bzw. RNA-Transkription hemmen. Die Substanzen können speziell über die Inhibierung des Enzyms Reverse Transkriptase oder über einen Kettenabbruch der wachsenden DNA-Kette die Vermehrung von Retroviren beeinflussen (vgl. Proc. Natl. Acad. Sci. USA 83, 1911, 1986 bzw. Nature 325, 773 1987). Von besonderem therapeutischem Interesse ist die Hemmwirkung auf das HIV-Virus, dem Verursacher der Immunschwäche-Erkrankung AIDS. Zur Behandlung von AIDS ist heute nur 3'-Azido-3'-desoxythymidin ( DE-A-3608606) bei AIDS Patienten zugelassen. Jedoch machen toxische Nebenwirkungen des 3'-Azido-3'-desoxythymidins auf das Knochenmark bei etwa 50 % der behandelten Patienten Bluttransfusionen erforderlich. Die Verbindungen der allgemeinen Formel I besitzen diese Nachteile nicht. Sie wirken antiviral, ohne in pharmakologisch relevanten Dosen cytotoxisch zu sein.

Die erfindungsgemäßen Substanzen der Formel I lassen sich auch vorteilhaft bei der DNA-Sequenzierung nach Sanger einsetzen. Besonders die Sequenzierung d(G-C)-reicher DNA-Fragmente wird durch die Bildung von Sekundärstrukuren, die zu einer Bandenkompression im Bereich von d(G-C)-Clustern führen, erschwert.

Die erfindungsgemäßen Verbindungen der Formel I, in denen $R^6$ und $R^7$ Wasserstoff bedeuten, werden bei der DNA-Sequenzierung nach Sanger als Kettenterminatoren anstelle der bekannten 2',3'-Didesoxyverbindungen verwendet.

Nucleinsäuren, die als Baustein eine oder mehrere Verbindungen der Formel I enthalten, können nach bekannten Verfahren hergestellt werden (beispielsweise Nucleic Acids Research Vol. 14, Nr. 5, 1986, S. 2319 ff). Sie entstehen aber auch beispielsweise bei der DNA-Sequenzierung. Wird als Baustein eine Verbindung der Formel I verwendet, in der $R^6$ Wasserstoff bedeutet, so kann ein solcher Baustein nur einmal, nämlich am Kettenende der Nucleinsäure eingebaut sein. Die erfindungsgemäßen Nucleinsäuren sind aus 2 bis 1000, vorzugsweise 8 bis 50 Nucleotidbausteinen aufgebaut. Besonders bevorzugt sind Nucleinsäuren mit 15 - 30 Nucleotidbausteinen.

Diese Nucleinsäuren können ebenfalls als antivirale Mittel eingesetzt werden. Als sogenannte antisense-Nucleinsäure hybridisiert diese Nucleinsäure mit der ss-DNA/RNA des Virus und erschwert die Transkription zur Virus-DNA. Solche Nucleinsäuren können insbesondere als Mittel gegen AIDS verwendet werden, da sie nicht oder nur schwer durch zelleigene Restriktionsenzyme abgebaut werden.

Zur Herstellung von Arzneimitteln werden die Substanzen der allgemeinen Formel I, ihre pharmakologisch verträglichen Salze oder sie enthaltende Nucleinsäuren in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die erfindungsgemäßen Substanzen können in flüssiger oder fester Form enteral oder parenteral appliziert werden. Als Injektionsmedium kommt vorzugsweise Wasser zur Anwendung, welches die bei Injektionslösungen üblichen Zusätze, wie Stabilisierungsmittel, Lösungsvermittler oder Puffer enthält.

Derartige Zusätze sind z.B. Tartrat- und Citratpuffer, Ethanol, Komplexbildner (wie Ethylendiamintetraessigsäure und deren nichttoxischen Salze) und hochmolekulare Polymere (wie flüssiges Polyethylenoxid) zur Viskositätsregulierung. Feste Trägerstoffe sind z.B. Starke, Lactose, Mannit, Methylcellulose, Talkum, hochdisperse Kieselsäuren, hochmolekulare Fettsäuren (wie Stearinsäure), Gelatine, Agar-Agar, Calciumphosphat, Magnesiumstearat, tierische und pflanzliche Fette und feste hochmolekulare Polymere (wie Polyethylenglykole). Für orale Applikation geeignete Zubereitungen können gewünschtenfalls Geschmacks- und Süßstoffe enthalten.

Die erfindungsgemäßen Verbindungen werden üblicherweise in Mengen von 0.1 - 100 mg, vorzugsweise 0.2 - 80 mg pro Tag und pro kg Körpergewicht appliziert.

Bevorzugt ist es, die Tagesdosis auf 2 - 5 Applikationen zu verteilen, wobei bei jeder Applikation 1 - 2 Tabletten mit einem Wirkstoffgehalt von 0.5 - 1000 mg verabreicht werden. Die Tabletten können auch retardiert sein, wodurch sich die Anzahl der Applikationen pro Tag auf 1 - 3 vermindert. Der Wirkstoffgehalt der retardierten Tabletten kann 2 - 2000 mg betragen. Der Wirkstoff kann auch durch Injektion einbis achtmal pro Tag bzw. durch Dauerinfusion gegeben werden, wobei Mengen von 5 - 4000 mg/Tag normalerweise ausreichen.

Neben den in den Beispielen genannten Verbindungen kommen im Sinne der vorliegenden Erfindung beispielsweise die folgenden Verbindungen in Frage:

1-(2,3-Didesoxy-β-D-glyceropentofuranosyl)-4,6-diamino-benzimidazol
1-(2,3-Didesoxy-β-D-glyceropentofüranosyl)-6-hydroxy-benzimidazol
1-(2,3-Didesoxy-β-D-glyceropentofuranosyl)-4-amino-6-hydroxy-benzimidazol
1-(2,3-Didesoxy-β-D-glyceropentofuranosyl)-4-methylamino-benzimidazol
1-(2,3-Didesoxy-β-D-glyceropentofuranosyl)-4,6-dihydroxy-benzimidazol
1-(2,3-Didesoxy-β-D-glyceropentofuranosyl)-4-hydroxy-6-amino-benzimidazol
1-(2,3-Didesoxy-β-D-glyceropentofuranosyl)-4-methyl-benzimidazol
1-(2,3-Didesoxy-2,3-didehydro-β-D-glyceropentofuranosyl)-4-amino-6-chlorbenzimidazol
1-(2,3-Didesoxy-2,3-didehydro-β-D-glyceropentofuranosyl)-4-mercaptobenzimidazol
1-(2,3-Didesoxy-2,3-didehydro-β-D-glyceropentofuranosyl)-4-methyl-mercaptobenzimidazol

1-(2,3-Didesoxy-2,3-didehydro-β-D-glyceropentofuranosyl)-4-methoxybenzimidazol
1-(2,3-Didesoxy-2,3-didehydro-β-D-glyceropentofuranosyl)-4-dimethylaminobenzimidazol
1-(2,3-Didesoxy-β-D-glyceropentofuranosyl)-4,6-diamino-1H-pyrrolo-[2,3-b]pyridin
1-(2,3-Didesoxy-β-D-glyceropentofuranosyl)-6-hydroxy-1H-pyrrolo-[2,3-b]pyridin
1-(2,3-Didesoxy-β-D-glyceropentofuranosyl)-4-amino-6-hydroxy-1H-pyrrolo[2,3-b]pyridin
1-(2,3-Didesoxy-β-D-glyceropentofuranosyl)-4-methylamino-1H-pyrrolo[2,3-b]pyridin
1-(2,3-Didesoxy-β-D-glyceropentofuranosyl)-4,6-dihydroxy-1H-pyrrolo[2,3-b]pyridin
1-(2,3-Didesoxy-β-D-glyceropentofuranosyl)-4-hydroxy-6-amino-1H-pyrrolo [2,3-b]pyridin
1-(2,3-Didesoxy-β-D-glyceropentofuranosyl)-4-methyl-1H-pyrrolo [2,3-b]pyridin
1-(2,3-Didesoxy-2,3-didehydro-β-D-glyceropentofuranosyl)-4-amino-6-chlor-1H-pyrrolo[2,3-b]pyridin
1-(2,3-Didesoxy-2,3-didehydro-β-D-glyceropentofuranosyl)-4-mercapto-1H-pyrrolo[2,3-b]pyridin
1-(2,3-Didesoxy-2,3-didehydro-β-D-glyceropentofüranosyl)-4-methyl-mercapto-1H-pyrrolo[2,3-b]pyridin
1-(2,3-Didesoxy-2,3-didehydro-β-D-glyceropentofuranosyl)-4-methoxy-1H-pyrrolo[2,3-b]pyridin
1-(2,3-Didesoxy-2,3-didehydro-β-D-glyceropentofuranosyl)-4-dimethylamino-1H-pyrrolo[2,3-b]pyridin
1-(2,3-Didesoxy-β-D-glyceropentofuranosyl)-4-amino-1H-imidazo[4,5-c]pyridin
1-(2,3-Didesoxy-β-D-glyceropentofuranosyl)-4,6-diamino-1H-imidazo [4,5-c]pyridin
1-(2,3-Didesoxy-β-D-glyceropentofuranosyl)-6-hydroxy-1H-imidazo[4,5-c]pyridin
1-(2,3-Didesoxy-β-D-glyceropentofuranosyl)-4-amino-6-hydroxy-1H-imidazo [4,5-c]pyridin
1-(2,3-Didesoxy-β-D-glyceropentofuranosyl)-4-methylamino-1H-imidazo [4,5-c]pyridin
1-(2,3-Didesoxy-β-D-glyceropentofuranosyl)-4,6-dihydroxy-1H-imidazo [4,5-c]pyridin
1-(2,3-Didesoxy-β-D-glyceropentofuranosyl)-4-hydroxy-6-amino-1H-imidazo [4,5-c]pyridin
1-(2,3-Didesoxy-β-D-glyceropentoturanosyl)-4-methyl-1H-imidazo [4,5-c]pyridin
1-(2,3-Didesoxy-2,3-didehydro-β-D-glyceropentofuranosyl)-4-amino-6-chlor-1H-imidazo[4,5-c]pyridin
1-(2,3-Didesoxy-2,3-didehydro-β-D-glyceropentofuranosyl)-4-mercapto-1H-imidazo[4,5-c]pyridin
1-(2,3-Didesoxy-2,3-didehydro-β-D-glyceropentofuranosyl)-4-methyl-mercapto-1H-imidazo[4,5-c]pyridin
1-(2,3-Didesoxy-2,3-didehydro-β-D-glyceropentofuranosyl)-4-methoxy-1H-imidazo[4,5-c]pyridin
1-(2,3-Didesoxy-2,3-didehydro-β-D-glyceropentofuranosyl)-4-dimethylamino-1H-imidazo[4,5-c]pyridin
1-(2,3-Didesoxy-β-D-glyceropentofuranosyl)-4-amino-1H-triazolo[4,5-d]pyrimidin
1-(2,3-Didesoxy-β-D-glyceropentofuranosyl)-4,6-diamino-1H-triazolo[4,5-d]pyrimidin
1-(2,3-Didesoxy-β-D-glyceropentofuranosyl)-6-hydroxy-1H-triazolo[4,5-d]pyrimidin
1-(2,3-Didesoxy-β-D-glyceropentofuranosyl)-4-amino-6-hydroxy-1H-triazolo[4,5-d]pyrimidin
1-(2,3-Didesoxy-β-D-glyceropentofuranosyl)-4-methylamino-1H-triazolo [4,5-d]pyrimidin
1-(2,3-Didesoxy-β-D-glyceropentofuranosyl)-4,6-dihydroxy-1H-triazolo [4,5-d]pyrimidin
1-(2,3-Didesoxy-β-D-glyceropentofuranosyl)-4-hydroxy-6-amino-1H-triazolo [4,5-d]pyrimidin
1-(2,3-Didesoxy-β-D-glyceropentofuranosyl)-4-methyl-1H-triazolo[4,5-d]pyrimidin
1-(2,3-Didesoxy-2,3-didehydro-β-D-glyceropentofuranosyl)-4-amino-6-chlor-1H-triazolo[4,5-d]pyrimidin
1-(2,3-Didesoxy-2,3-didehydro-β-D-glyceropentofuranosyl)-4-mercapto-1H-triazolo[4,5-d]pyrimidin
1-(2,3-Didesoxy-2,3-didehydro-β-D-glyceropentofuranosyl)-4-methyl-mercapto-1H-triazolo[4,5-d]pyrimidin
1-(2,3-Didesoxy-2,3-didehydro-β-D-glyceropentofuranosyl)-4-methoxy-1H-triazolo[4,5-d]pyrimidin
1-(2,3-Didesoxy-2,3-didehydro-β-D-glyceropentofuranosyl)-4-dimethylamino-1H-triazolo[4,5-d]pyrimidin
8-(2,3-Didesoxy-β-D-glyceropentofuranosyl)-8H-imidazo[1,2-a]-s-triazin-4-on
8-(2,3-Didesoxy-β-D-glyceropentofuranosyl)-2-amino-imidazo[1,2-a]-s-triazin-4-on
8-(2,3-Didesoxy-β-D-glyceropentofuranosyl)-2-hydroxy-imidazolo[1,2-a]-s-triazin-4-on
1-(2,3-Didesoxy-2,3-didehydro-β-D-glyceropentofuranosyl)-8H-imidazo [1,2-a]-s-triazin-4-on
1-(2,3-Didesoxy-2,3-didehydro-β-D-glyceropentofuranosyl)-2-chloro-8H-imidazo[1,2-a]-s-triazin-4-on
Die Erfindung wird durch die folgenden Beispiele näher erläutert:

Vergleichsbeispiel

1-(2,3-Didesoxy-β-D-glyceropentofuranosyl)-4-nitro-1H-indol

a) 5-O-[(1,1-Dimethylethyl)dimethylsilyl]-(2,3-didesoxy-α,β-D-glyceropentofuranosyl)chlorid
5- O- [(1,1-Dimethylethyl)dimethylsilyl)-2,3-didesoxy-▪,β-D-glyceropentofuranose (1.51 g, 6.5 mmol) [M. Okabe, R.-C. Sun, S. Y.-K. Tam, L.T. Todaro, and D. L. Coffen , J. Org. Chem. 53, 4780, 1988] werden in 26 ml Tetrahydrofuran gelöst und mit $CCl_4$ (1 ml) unter $N_2$ versetzt. Man kühlt auf -80°C und versetzt während 15 min tropfenweise mit Tris-(dimethylamino)phosphin (1.56 ml). Nach ca. 2 h werden nochmals die gleichen Mengen $CCl_4$ und Phosphin hinzugefügt. Nach 6h zeigt das DC (Kieselgel, EtOAc-Petrolether, 2:8) einen etwa 50 proz. Umsatz des Lactols an. Die kalte Reaktionslösung der

Halogenose wird direkt in die vorbereitete Glycosylierungsreaktion eingetragen.

b) 1-(2,3-Didesoxy-5-O-(tert-butyldimethylsilyl)-D-glyceropentofuranosyl)-4-nitro-1H-indol

1.0 g (6.16 mmol) 4-Nitroindol wird in 200 ml MeCN gelöst und zusammen mit 690 mg (12.32 mmol) KOH und TDA-1 20 Min. bei RT gerührt. Die in situ bereitete kalte Lösung der unter a) erhaltenen Halogenose (aus 12.32 mmol Lactol) wird der Suspension portionsweise zugespritzt und die Reaktionsmischung weitere 45 Min. intensiv gerührt. Unlösliche Bestandteile werden abfiltriert und das Filtrat zur Trockene eingedampft. Man adsorbiert an Kieselgel 60 (10 g) und chromatographiert an Kieselgel 60 H (Petrolether/EtOAc 8:2). Eindampfen der Hauptzone liefert ein Anomerengemisch in 60% Ausbeute, das aus 30% des $\beta$-Anomeren und 30% des $\alpha$:-Anomeren besteht.

$^1$H-NMR ([$D_6$]DMSO): d ($\beta$-Anomer: 4.16 (m, 1H, H-4'), 6.42 (dd, J = 3.5 Hz und J = 6.4 Hz, 1H, H-1'); ▪-Anomer: 4.32 (m, 1H, H-4'), 6.47 (dd, H-1').

| | | | | | | | |
|---|---|---|---|---|---|---|---|
| $C_{19}H_{28}N_2O_4Si$ (376.53) | Ber.<br>Gef. | C<br>C | 60.61<br>60.77 | H<br>H | 7.50<br>7.42 | N<br>N | 7.44<br>7.32 |

c) 1-(2,3-Didesoxy-$\beta$D-glycero-pentofuranosyl)-4-nitro-1H-indol

Das unter b) erhaltene $\beta$-Anomere wird in THF gelöst. Nach Zugabe von $Bu_4NF$ (1M Lösung in THF) rührt man 30 Min. bei Raumtemperatur. Das Lösungsmittel wird im Vakuum verdampft und der Rückstand an Kieselgel 60 adsorbiert. Nach Säulenchromatographie ($CH_2Cl_2$ - MeOH, 97:3) erhält man die Titelverbindung als gelbes Öl. $R_f$($CH_2Cl_2$ - MeOH, 97:3) = 0.4.

$^1$H-NMR ([$D_6$]DMSO): d = 4.11 (m, 1H, H-4'), 4.89 (t, J = 5.4 Hz, 1H, 5'-OH), 6.41 (dd, J = 4.0 und 6.7 Hz, 1H, H-1'), 7.09 (d, J = 3.2 Hz, 1H, H-3), 7.37 (pt, J = 8.1 Hz, 1H, H-6), 8.04 (d, J = 3.2 Hz, 1H, H-2).

## Beispiel 1

### 1-(2,3-Didesoxy-$\beta$-D-glyceropentofuranosyl)benzimidazol

a) 1-[2-Desoxy-3,5-di-O-(4-methylbenzoyl)-$\beta$-D-erythropentofuranosyl]benzimidazol

Die Glycosylierung von Benzimidazol mit 2-Desoxy-3,5-di-O-(p-toluoyl)-$\alpha$-D-erythro-pentoturanosylchlorid erfolgte unter den gleichen Bedingungen wie im Vergleichsbeispiel unter b) beschrieben. Daten siehe [Z. Kazimierczuk, R. Stolarski und D. Shugar, Z. Naturforsch. 40c, 715 (1985)].

b) 1-(2-Desoxy-$\beta$-D-erythro-pentofuranosyl)benzimidazol

Die Herstellung dieser Substanz erfolgt aus der unter a) erhaltenen Verbindung durch Detoluoylierung. Lit. wie unter a)

c) 1-[2-Desoxy-5-O-(tert-butyldimethylsilyl)-$\beta$-D-erythropento-furanosyl] benzimidazol

1.0 g (4.26 mmol) der unter b) erhaltenen Verbindung wird 2x mit Pyridin abgedampft und anschließend in 20 ml Pyridin gelöst. In der Kälte (0°C) werden 1.3 ml (1.391 g, 5.06 mmol) TBDPSiCl hinzugefügt und die Lösung 30 min. bei 0 C gerührt. Man läßt langsam auf Raumtemperatur erwärmen und rührt weitere 24 h.

Chromatographie (Säule 25 x 4 cm/ Kieselgel 60) liefert ein farbloses Öl; Behandlung mit Diethylether ergibt farblose Kristalle vom Schmp. 144-145°C, Rf($CHCl_3$/MeOH 9:1) = 0.45. Ausbeute: 1.24g (61%). UV (MeOH): $\lambda_{max}$ (e) = 246 (7500), 252 (Sch, 7200), 265 (4800), 273 (4900), 281 (4200).

$^1$H NMR ([$D_6$]DMSO): d = 0.94 (s, 9H, 3 $CH_3$), 2.35 (m, 1H, H-2'b), 2.65 (m, 1H, H-2'a), 3.77 (m, 2H, H-5'), 3.94 (m, 1H, H-4'), 4.49 (m, 1H, H-3'), 5.44 (d, J = 4.1 Hz, 1H, 3'-OH), 6.37 (pt, J = 6.5 Hz, 1H, H-1'), 7.10-7.66 (m, 14 aromat. H), 8.36 (s, 1H, H-2).

| | | | | | | | |
|---|---|---|---|---|---|---|---|
| $C_{28}H_{32}N_2O_3Si$ (472.66) | Ber.<br>Gef. | C | 71.15<br>71.23 | H | 6.82<br>6.85 | N | 5.93<br>5.94 |

d) 1-[2-Desoxy-5-O-(tert-butyldimethylsilyl)-3-O-phenoxythio-carbonyl-$\beta$-D-erythro-pentofuranosyl]-benzimidazol

500 mg (1.06 mmol) der unter c) erhaltenen Verbindung in wasserfreiem MeCN (20 ml) werden mit 4-(Dimethylaminopyridin (DMAP, 768 mg, 6.36 mmol) und Phenoxythiocarbonylchlorid (PTC-Cl, 320 ml, 2.34 mmol) 16 h bei RT gerührt. Nach Abdampfen des LM chromatographiert man an Kieselgel 60 H und erhält aus der Hauptzone einen farblosen Schaum (480 mg, 74%). Rf ($CH_2Cl_2$/Aceton, 95:5) = 0.5. UV (MeOH): $\lambda_{max}$ (e) = 243 (12000), 264 (Sch, 5100), 273 (4800), 280 (4100).

$^1H$ NMR ($[D_6]DMSO$): d = 1.03 (s, 9H, 3 $CH_3$), 2.88 (m, 1H, H-2'b), 3.06 (m, 1H, H-2'a), 3.99 (m, 2H, H-5'), 4.48 (m, 1H, H-3'), 6.55 (dd, J = 5.4 und 9.0 Hz, 1H, H-1'), 7.09-7.84 (m, 19 aromat. H), 8.46 (s, 1H, H-2).

| | | | | | | | | |
|---|---|---|---|---|---|---|---|---|
| $C_{35}H_{36}N_2O_4SSi$ (608.84) | C | 69.05<br>69.26 | H | 5.96<br>5.99 | N | 4.60<br>4.74 | S | 5.27<br>5.12 |

e) 1-[2,3-Didesoxy-5-O-(tert-butyldimethylsilyl)-$\beta$-D-glyceropentofuranosyl)benzimidazol
Eine Lösung von 350 mg (0.57 mmol) der unter d) erhaltenen Verbindung in wasserfreiem Toluol (20 ml) wird in Gegenwart von AIBN (35 mg) und Tributylzinnhydrid (200 ml) 3h bei 80°C unter Argonatmosphäre gerührt. Chromatographische Aufarbeitung. Ausbeute 83%.
Rf($CH_2Cl_2$/Aceton) = 0.4. UV (MeOH): $\lambda_{max}$ (e) = 247 (7300), 251 (Sch, 7100), 265 (4600), 273 (4700), 281 (4100). $^1H$ NMR ($[D_6]DMSO$): d = 0.96 (s, 9H, 3 $CH_3$), 2.13 (m, 2H, H-3'), 2.47 (m, 2H, H-2'), 3.74 (m, 2H, H-5'), 4.26 (m, 1H, H-4'), 6.32 (pt, J = 4.8 Hz, 1H, H-1'), 7.22-7.71 (m, 14 aromat. H), 8.42 (s, 1H, H-2)

| | | | | | | | |
|---|---|---|---|---|---|---|---|
| $C_{28}H_{32}N_2O_2Si$ (456.66) | Ber.<br>Gef. | C | 73.65<br>73.64 | H | 7.06<br>7.02 | N | 6.13<br>6.10 |

f) 1-(2,3-Didesoxy-$\beta$-D-glyceropentofuranosyl)benzimidazol
Die unter e) erhaltene Verbindung (200 mg) wird in THF gelöst, mit $Bu_4NF$ (1M Lsg. in THF) versetzt und 30 Min. bei RT gerührt. Abdampfen des Lösungsmittels und chromatographische Aufarbeitung liefert ein farbloses Öl (Ausbeute 69%).
$^1H$ NMR ($[D_6]DMSO$): d = 2.06 (m, 2H, H-3'), 2.36 (m, 2H, H-2'), 3.55 (m, 2H, H-5'), 4.14 (m, 1H, H-4'), 4.93 (m, 1H, 5'-OH), 6.28 (dd, J = 4.1 und 6.5 Hz, 1H, H-1'), 7.27 (m, 2H, H-5 und H- 6), 7.68 (m, 2H, H-4 und H-7), 8.51 (s, 1H, H-2).

## Beispiel 2

1-(2,3-Didesoxy-$\beta$-D-glyceropentofuranosyl)benzimidazol

a) Die in Beispiel 1 beschriebene Titelverbindung erhält man auch analog Vergleichsbeispiel b), indem man 1.0 g (8.48 mmol) Benzimidazol in 200 ml MeCN löst und mit KOH (1.9g) und TDA-1 (0.8 mmol) 15 min. bei RT rührt. Die in situ bereitete kalte Lösung der Halogenose (bereitet aus 17 mmol Lactol) wird der Mischung portionsweise zugespritzt. Man rührt weitere 30 Min. bei RT, filtriert und verdampft das LM im Vakuum. Der Rückstand wird an Kieselgel 60 chromatographiert. Man erhält das $\alpha$-Anomer in 30% und das $\beta$-Anomer in 30% Ausbeute.
$^1H$ NMR ($[D_6]DMSO$): $\beta$-Anomer: d 4.17 (m, 1H, H-4'), 6.26 (dd, 1H, H-1'), 8.42 (s, 1H, H-2); $\alpha$-Anomer: 4.34 (m, 1H, H-4'), 6.33 (pt, 1H, H-1'), 8.38 (s, 1H, H-2).
b) Nach Abspaltung der Silylschutzgruppe analog Vergleichsbeispiel c) erhält man die Titelverbindung.

## Beispiel 3

1-(2,3-Didesoxy-$\beta$-D-glyceropentofuranosyl)-1H-pyrrolo[2,3-b]pyridin

a) 1-[2'-Desoxy-5-O-(triphenylmethyl)-$\beta$-D-erythro-pentofuranosyl]-1H-pyrrolo[2,3-b]pyridin
1.48 g (6.3 mmol) 1-(2-Desoxy-$\beta$-D-erythro-pentofuranosyl)-1H-pyrrolo[2,3-b]pyridin [F. Seela und R. Gumbiowski, Heterocycles, 29, 795 (1989)] wird zweimal mit je 30 ml trockenem Pyridin abgedampft und in 40 ml Pyridin gelöst. Anschließend gibt man unter Argon 3.55 g (12.6 mmol) Triphenylmethylchlorid sowie 3.3 ml (19 mmol) Hünigbase hinzu und rührt 4 h bei Raumtemperatur.

Das Reaktionsgemisch wird in 200 ml 5proz. wässerige $NaHCO_3$ gegeben und dreimal mit je 150 ml $CH_2Cl_2$ extrahiert. Die vereinigten organischen Phasen werden mit $Na_2SO_4$ getrocknet, filtriert und an Kieselgel 60H (Säule 7 x 4.5 cm, $CH_2Cl_2$/Aceton, 8:2) chromatographiert. Nach dem Eindampfen der Hauptzone erhält man einen farblosen Schaum; 1.75 g (58%). DC (Kieselgel, $CH_2Cl_2$/Aceton, 8:2) Rf 0.8. UV (MeOH): $\lambda_{max}$ = 288 um (e = 7600).
$^1H$-NMR ($[D_6]DMSO$): d = 2.28 (m, 2'-Ha); 2.61 (m, 2'-Hb); 3.17 (d, J = 4.8 Hz, 5'-H2); 3.97 (m, 4'-H);

4.39 (m, 4'-H); 5.38 (d, J = 4.7 Hz, 3'-OH); 6.53 (d, J = 3.6 Hz, 3'-H); 6.76 (pt, J = 4.0 Hz, 1'-H); 7.13 (dd, J = 4.7 Hz, J = 7.8 Hz, 5-H); 7.59 (d, J = 3.7 Hz, 2-H); 7.97 (dd, J = 1.5 Hz, J = 7.8 Hz, 4-H); 8.24 (dd, J = 1.5 Hz, J = 4.7 Hz, 6-H).

| | | | | | | | |
|---|---|---|---|---|---|---|---|
| $C_{31}H_{28}N_2O_3$ (476.58) | Ber. | C | 78.13 | H | 5.92 | N | 5.88 |
| | Gef. | C | 78.06 | H | 6.04 | N | 5.79 |

b) 1-(2-Deoxy-5-O-triphenylmethyl-3-O-phenoxythiocarbonyl-β-D-erythro-pentofuranosyl)-1H-pyrrolo[2,3-b]pyridin

Eine Lösung von 1.75 g (3.7 mmol) der unter a) erhaltenen Verbindung in 50 ml abs. Acetonitril wird unter Argon mit 1.12 g (9.2 mmol) 4-(Dimethylamino)pyridin und 1.0 ml (7.4 mmol) Phenoxythiocarbonylchlorid versetzt und 48 h bei Raumtemperatur gerührt. Nach dem Eindampfen der Reaktionsmischung wird der Rückstand an Kieselgel 60 H (Säule 10x4 cm, $CH_2Cl_2$) chromathographiert. Aus der Hauptzone ergeben sich 1.2 g (51%) eines farblosen Schaums. - UV (MeOH): $\lambda_{max}$ = 285 nm ($\epsilon$ = 8600).

$^1$H-NMR ([$D_6$]DMSO): d = 2.77 (m, 2'-Ha); 3.18 (m, 2'-Hb) 3.38 (m, 5'-H2); 4.42 (m, 4'-H), 5.93 (m, 3'-H); 6.62(d, J = 3.7 Hz, 3-H); 6.81 (dd, J = 5.5 Hz, J = 9.1 Hz, 1'-H); 7.18 (dd, J = 4.7 Hz, J = 7.8 Hz 5-H); 7.67 (d, J = 3.7 Hz, 2-H); 8.03 (dd, J = 1.4 Hz, J = 7.8 Hz, 4-H) 8.24 (dd, J = 1.4 Hz, J = 4.7 Hz, 6-H) und andere aromatische Protonen.

| | | | | | | | |
|---|---|---|---|---|---|---|---|
| $C_{38}H_{32}N_2O_4S$ (612.74) | Ber. | C | 74.49 | H | 5.26 | N | 4.57 |
| | Gef. | C | 74.65 | H | 5.41 | N | 4.35 |

c) 1-(2,3-Didesoxy-5-O-triphenylmethyl-β-D-glycero-pentofuranosyl)-1H-pyrrolo[2,3-b]pyridin

Eine Lösung von 1.2 g (1.86 mmol) der unter b) erhaltenen Verbindung in 60 ml absolutem Toluol wird unter Argon mit 90 mg (0.6 mmol) AIBN und 1.1 ml (4 mmol) Tributylzinnhydrid versetzt und 5h bei 80°C gerührt. Nach dem Eindampfen der Reaktionsmischung wird der Rückstand an Kieselgel 60 H (Säule 8 x 3 cm, $CH_2Cl_2$) chromathographiert. Aus der Hauptzone ergeben sich 0.8 g (93%) amorphes Produkt. Umkristallisation aus iPrOH liefert, farblose Nadeln vom Schmp. 115°C.

DC (Kieselgel, $CH_2Cl_2$/Aceton, 95 : 5), Rf: 0.83 - UV (MeOH): $\lambda_{max}$ = 289 nm ($\epsilon$ = 8100).

$^1$H-NMR ([D6]DMSO): d = 2.08 (m, 3'-H2); 2,37 (m, 2'-H2); 3.12 (m, 5'-H); 4.24 (m, 4'-H); 6.48 (d, J = 3.7 Hz, 3-H); 6.63 (dd, J = 4.0 Hz, J = 6.9 Hz, 1'-H); 7.13 (dd J = 4.7 Hz, J = 7.8 Hz, 5-H), 7.61 (d; J = 3.7 Hz, 2-H), 7.97 (dd, J = 1.5 Hz, J = 7.8 Hz, 4-H); 7.26 (dd, J = 1.5 Hz, J = 4.7 Hz, 6-H) und andere aromatische Protonen.

| | | | | | | | |
|---|---|---|---|---|---|---|---|
| $C_{31}H_{28}N_2O_2$ (460.58) | Ber. | C | 80.84 | H | 6.13 | N | 6.08 |
| | Gef. | C | 80.79 | H | 6.16 | N | 6.14 |

d) 1-(2,3-Didesoxy-β-D-glycero-pentofuranosyl)-1H-pyrrolo[2,3-b]pyridin

240 mg (0.52 mmol) der unter c) erhaltenen Verbindung werden mit 30 ml 80proz. Essigsäure versetzt und 3 h bei Raumtemperatur gerührt. Das Lösungsmittel wird im Ölpumpenvakuum abgezogen und der Rückstand mehrfach mit Wasser nachgedampft. Der Rückstand wird an Kieselgel 60 H (Säule 8 x 3 cm, $CH_2Cl_2$/Ethylacetat, 95 :5) chromatographiert. Der Rückstand der Hauptzone wird aus Wasser umkristallisiert. 102 mg (90%) farblose Kristalle vom Schmp. 124-125°C. - UV (MeOH): $\lambda_{max}$ = 288 um ($\epsilon$ = 7500).

$^1$H-NMR ([$D_6$]DMSO): d = 2.08 (m, 3'-H2); 2.31 (m, 2'-H2); 3.56 (m, 5'-H2); 4.08 (m, 4'-H); 4.98 (tr, J = 5.5 Hz, 5'-OH); 6.54 (d, J = 3.2 Hz, 3-H); 6.59 (pt, J = 5.4Hz, 1'-H); 7.13 (dd, J = 4.7 Hz, J = 7.7 Hz, 5-H); 7.77 (d, 1H, J = 3.2 Hz, 2-H); 7.98 (d, J = 7.7 Hz, 4-H); 8.25 (d, J = 4.8 Hz, 6-H).

| | | | | | | | |
|---|---|---|---|---|---|---|---|
| $C_{12}H_{14}N_2O_2$ (218.26) | Ber. | C | 66.04 | H | 6.47 | N | 12.83 |
| | Gef. | C | 66.09 | H | 6.57 | N | 12.84 |

Beispiel 4

1-(2,3-Didesoxy-$\beta$-D-glyceropent-2-enofuranosyl)-4-nitro-1H-pyrrolo[2,3-b]pyridin

a) 1-[5-O-((1,1-Dimethylethyl)-diphenylsilyl))-(2'-desoxy-$\beta$-D-erythro-pentofuranosyl)]-4-nitro-1H-pyrrolo-[2,3-b]pyridin

1.0 g (3.6 mmol) 1-(2-Desoxy-$\beta$-D-erythro-pentofuranosyl)-4-nitro-1H-pyrrolo[2,3-b]pyridin [F. Seela und R. Gumbiowski, Heterocycles, 29, 795 (1989)] wird zweimal mit je 30 ml trockenem Pyridin abgedampft, in 50 ml Pyridin gelöst und unter $N_2$ auf 0°C gekühlt. Nun werden 1.18 ml (4.6 mmol) 1,1-Dimethylethyldiphenylsilylchlorid durch ein Septum eingespritzt. Anschließend entfernt man das Kältebad und läßt 8 h bei Raumtemperatur rühren. Die Reaktionsmischung wird eingedampft, der Rückstand mit 200 ml $CHCl_3$ aufgenommen, zweimal mit je 40 ml 0.1 N HCl und anschließend mit wenig Wasser gewaschen und über $Na_2SO_4$ getrocknet. Das Lösemittel wird abgezogen und das zurückbleibende gelbe Ol wird an Kieselgel 60 (Säule 10 x 3 cm, $CH_2Cl_2$/Aceton, 95:5) chromatographiert. Aus der Hauptzone erhält man beim Abdamfen 1.5g (81 %) eines gelben Schaums. - UV (MeOH): $\lambda_{max}$ = 357, 338 um ($\epsilon$ = 4400, 4400).

$^1$H-NMR ([$D_6$]DMSO): d = 0.99 (s, $CH_3$); 2.38 (m, 2'-Hb); 2.63 (m, 2'-Ha); 3.75 (dd, J = 4.8 Hz, 10.9 Hz, 5'-H); 3.87 (dd, J = 4.8 Hz, 10.9 Hz, 5'-H); 3.96 (m, 4'-H); 4.53 (m, 3'-H); 5.46 (d, J = 4.4 Hz, 3'-OH); 6.79 (tr, J = 6.6 Hz, 1'-H); 6.98 (d, J = 3.6 Hz, 3-H); 7.97 (d, J = 5.3 Hz, 5-H); 8.07 (d, J = 3.6 Hz, 2-H); 8.53 (d, J = 5.3 Hz, 6-H) und andere arom. Protonen.

| | | | | | | | |
|---|---|---|---|---|---|---|---|
| $C_{28}H_{31}H_3O_5Si$ (517.66) | Ber. | C | 64.97 | H | 6.04 | N | 8.12 |
| | Gef. | C | 65.00 | H | 6.24 | N | 8.01 |

b) 1-[2-Desoxy-5-O-((1,1-dimethylethyl)-diphenylsilyl)-3-O-methylsulfonyl-($\beta$-D-erythro-pentofuranosyl)]-4-nitro-1H-pyrrolo[2,3-b]pyridin

Zu einer Lösung von 1.0 g (1.9 mmol) der unter a) erhaltenen Verbindung in 50 ml $CH_2Cl_2$ gibt man 1 ml (13 mmol) Methansulfonsäurechlorid und 15 ml Pyridin und läßt 12 h Rühren. Nach Beendigung der Reaktion (DC-Kontrolle) fügt man 20 ml Methanol zu und rührt weitere 15 min. Die Reaktionsmischung wird eingedampft, der Rückstand wird mit 200 ml $CHCl_3$ versetzt, zweimal mit je 40 ml 0.1 N HCl und anschließend mit wenig Wasser gewaschen und über $Na_2SO_4$ getrocknet. Das Lösemittel wird abgezogen und das zurückbleibende gelbe Öl an Kieselgel 60 (Säule 8 x 3 cm, $CH_2Cl_2$) chromatographiert. Aus dem Eindampfrückstand der Hauptzone erhält hat eine farblose Substanz die beim Abdampfen aufschäumt. 1.04 g (90 %) - UV (MeOH): $\lambda_{max}$ = 352, 338 nm ($\epsilon$ = 4400, 4700).

$^1$H-HMR ([D6]DMSO): d = 0.99 (s, $CH_3$); 2.80 (m, 2'-Ha); 3.11 (m, 2'-Hb); 3.90 (m, 5'-H2); 4.32 (m, 4'-H); 5.54 (m, 3'-H); 6.28 (pt, J = 6.3 Hz, 1'-H); 7.02 (d, J = 4.9 Hz, 3-H); 7.98 (d, J = 5.3 Hz, 5-H); 8.09 (d, J = 4.9 Hz, 2-H); 8.52(d, J = 5.3 Hz, 6-H) und andere arom. Protonen.

| | | | | | | | |
|---|---|---|---|---|---|---|---|
| $C_{29}H_{33}N_3O_7SSi$ (595.74) | Ber. | C | 58.47 | H | 5.58 | N | 7.03 |
| | Gef. | C | 58.69 | H | 5.65 | N | 7.03 |

c) 1-(2,3-Didesoxy-$\beta$-D-glyceropent-2-enofuranosyl)-4-nitro-1H-pyrrolo[2,3-b]pyridin

Zu einer Lösung von 400 mg (0.67 mmol) der unter b) erhaltenen Verbindung in 10 ml THF gibt man 5 ml 1 M Tetrabutylammoniumfluorid in THF und läßt 4 h unter Rückfluß rühren. Das Lösungsmittel wird im Vakuum abgezogen und das Rohprodukt an Kieselgel 60 (Säule 10 x 3 cm, $CH_2Cl_2$/MeOH; 95 : 5) chromathographiert. Der Abdampfrückstand wird aus Isopropanol kristallisiert. 120 mg (68%) gelbe Nadeln vom Schmp. 154°C. - UV (MeOH): $\lambda_{max}$ = 358, 338 nm ($\epsilon$ = 5000, 4700).

$^1$H-NMR ([$D_6$]DMSO): d = 3.57 (m, 5'-H2); 4.87 (m, 4'-H); 4.96 (tr, J = 5.4 Hz, 5'-OH); 6.14 (m, 2'-H); 6.52 (m, 3'-H); 7.05 (d, J = 3.6 Hz, 3-H); 7.40 (m, 1'-H); 7.99 (d, J = 5.3 Hz, 5-H); 8.03 (d, J = 3.6 Hz, 2-H); 8.58 (d, J = 5.3 Hz, 6-H).

| | | | | | | | |
|---|---|---|---|---|---|---|---|
| $C_{12}H_{11}N_3O_4$ (261.24) | Ber. | C | 55.17 | H | 4.24 | N | 16.09 |
| | Gef. | C | 55.27 | H | 4.38 | N | 16.03 |

Beispiel 5

4-Amino-1-(2,3-didesoxy-$\beta$-D-glycero-pentofuranosyl)-1H-pyrrolo[2,3-b]pyridin

50 mg der unter Beispiel 4c erhaltenen Verbindung werden in 30 ml Methanol gelöst und mit 0.1 ml Pyridin und 10 mg Pd/C (10% Pd) versetzt. Es wird zwei Stunden bei Raumtemperatur unter Normaldruck hydriert. Durch Entfärben der Lösung wir der Endpunkt angezeigt. Der Katalysator wird abfiltriert, mehrfach mit Methanol gewaschen, das Filtrat eingeengt und der Rückstand an Kieselgel 60 (Säule 8 x 1.5 cm, $CH_2Cl_2$/MeOH, 9 : 1) chromathographiert. Aus der Hauptzone erhält man 10 mg (22%) der farblosen Titelverbindung.
$^1$H-NMR ([$D_6$]DMSO): d = 1.99 (m, 3'-H2); 2.27 (m, 2'-H2); 3.50 (m, 5'-H2); 4.03 (m, 4'-H); 6.16 (d, J = 5.4 Hz, 5-H); 6.25 (s, $NH_2$); 6.34 (pt, J = 5.98 Hz, 1'-H); 6.52(d, J = 3.6 Hz, 3-H); 7.28 (d, J = 3.6 Hz, 2-H); 7.69 (d, J = 5.4 Hz, 6-H).

Beispiel 6

1-(2,3-Didesoxy-$\beta$-D-glycero-pentofuranosyl)-1H-pyrrolo[2,3-b]pyridin 5'-Triphosphat, Trimethylammoniumsalz

21,8 mg (0.1 mmol) der in Beispiel 3d erhaltenen Verbindung werden in 250 ml (2.14 mmol) Trimethylphosphat gelöst und mit 11.7 ml (0.13 mmol) frisch destilliertem $POCl_3$ bei 4°C versetzt. Nach 1.5h Rühren bei 4°C wird eine Lösung aus 0.5 mmol Bis(-tri-n-butylammonium)pyrophosphat in 1 ml DMF und 100 ml (0.42 mmol) Tri-n-butylamin hinzugegeben. Nach 1 min wird mit 1 M wässeriger Triethylammonium-bicarbonatlösung (TBK) neutralisiert und anschließend das Lösungsmittel im Vakuum abgezogen. Der Rückstand wird in 150 ml Wasser aufgenommen und an Fraktogel TSK (Säule: 30 x 2.6 cm) adsorbiert. Gradientenelution (360 ml $H_2O$/ 360 ml 0.5 M TBK-Lösung) führ: bei 0.49 M TBK zu einer Hauptzone, aus der nach Abdampfen des Lösungsmittels 370 $A_{288}$-Einheiten (49%) eines farblosen amorphen Triphosphats in Form des Triethylammoniumsalzes erhalten werden. DC (2-Propanol/$NH_3$/$H_2O$ = 3 :1 :1), Rf = 0.12; HPLC (LiChrosorb RP-18; 0.1 M Ammoniumacetat/ 50% Acetonitril; 1 ml/min); Rt = 1.95 min; UV (MeOH): $\lambda_{max}$ = 288 um ($\epsilon$ = 7500).
$^{31}$P-NMR ($D_2O$; 0.1 M Tris- HCl (1:1), pH 7.0, 100 mM EDTA): d = -10.32 (d, J = 19 Hz, Pa); -22.04 (t, J = 19 Hz, P$\beta$); -7.46 (d, J = 19 Hz, Pgamma).

Beispiel 7

1-(2,3-Didesoxy-$\beta$-D-glyceropent-2-enofuranosyl)-4-nitro-1H-pyrrolo [2,3-b]pyridin 5'-Triphosphat, Triethylammoniumsalz

26 mg (0.1 mmol) der unter Beispiel 4c erhaltenen Verbindung wurden wie in Beispiel 6 angegeben phosphoryliert und aufgearbeitet. DC (2-Propanol/$NH_3$ $H_2O$; 3:1:1); Rf = 0.12. HPLC (LiChrosorb RP-18; 0.1 M Ammoniumacetat 50% Acetonitril; 1 ml/min); Rt = 1.91 min; Ausbeute 180 $A_{358}$-Einheiten (36%) farbloses amorphes Produkt. UV (MeOH): $\lambda_{max}$ = 358 um ($\epsilon$ = 5000).
$^{31}$P-NMR ($D_2O$, 0.1 M TRIS-HCl (1:1), pH 7, 100 mM EDTA) : d = -10.52 (d, J = 20 Hz, Pa); -21.75 (t, J = 20 Hz, P$\beta$); -6.18 (d, J = 20 Hz, Pgamma).

Beispiel 8

8-(2,3-Didesoxy-$\alpha$,($\beta$)-D-glyceropentofuranosyl)-8H-imidazo[1,2-a]-s-triazin-4-on

a) 8-{5-0-[(1,1-Dimethylethyl)dimethylsilyl]-2,3-dideoxy-$\alpha$,($\beta$)-D-glyceropentofuranosyl}-2-[(2-methylpropionyl)amino]-8H-imidazo[1,2-a]-s-triazin-4-on
2-[(2-Methylpropionyl)amino]-8H-imidazo[1,2-a]-s-triazin-4-on (500 mg, 2.26 mmol) werden in trockenem MeCN (100 ml) unter leichtem Erwärmen gelöst und mit $K_2CO_3$ (1 g) versetzt. Man fügt TDA-1 (50 ml) hinzu und läßt 10 min bei RT rühren. Anschließend wird die kalte Lösung des nach dem Vergleichsbeispiel unter a) hergestellten 5-0-[1,1-Dimethylethyl)dimethyl-silyl]-2,3-dideoxy-D-glycero-pentofuranosylchlorids in acht gleichen Portionen im Abstand von 2-3 min hinzugefügt und die Reaktionsmischung weitere 30 min bei RT unter $N_2$ gerührt. Dünnschichtchromatographische Reaktionskontrolle (Kieselgel, $CH_2Cl_2$-MeOH, 9:1) zeigt eine vollständige Umsetzung zu zwei Produkten an. Nach Filtration durch Celit

(1 cm) wird im Ölvakuum eingedampft (20-25°C) und der Rückstand sofort an Kieselgel 60H (Säule: 6 x 25 cm, $CH_2Cl_2$-MeOH, 95:5) chromatographiert. Aus der schnell wandernden Hauptzone erhält man das Anomerengemisch, das in $CH_2Cl_2$-MeOH 99:1 gelöst und erneut an Kieselgel 60H (Säule: 6 x 15 cm, $CH_2Cl_2$-MeOH 99:1 (1 l), $CH_2Cl_2$-MeOH 97:3 (2 l) chromatographiert wird. Aus der schneller wandernden Zone ($R_f$ (Kieselgel, $CH_2Cl_2$-MeOH 9:1) 0.90) erhält man nach Eindampfen 250 mg (25 %) des $\beta$-Anomeren als farblosen Schaum. $^1$H-NMR ([$D_6$]DMSO): 10.33 (s, NH); 7.64 (d, J = 2.7 Hz, H-7); 7.58 (d, J = 2.7 Hz, H-6); 6.14 (pt, J = 3.8 Hz, H-1'); 4.14 (m, H-4'); 3.75 (m, $H_2$-5'); 2.92 (m, CH); 2.40 (m, $H_2$-2'); 2.06 (m, $H_2$-3'); 1.07 und 1.05 (2 s, iBu-Me); 0.84 (s, t-Bu-Me); 0.01 (s, Si-Me). Aus der langsamer wandernden Zone ($R_f$ (Kieselgel, $CH_2Cl_2$-MeOH 9:1) 0.85) erhält man nach Eindampfen 270 mg (27 %) des a-Anomeren als farblosen Schaum. $^1$H-NMR ([$D_6$]DMSO): 10.3 (s, NH); 7.66 (d, J = 2.7 Hz, H-7); 7.59 (d, J = 2.7 Hz, H-6); 6.19 (dd, J = 5.8 Hz, H-1'); 4.51 (m, H-4'); 3.62 (m, $H_2$-5'); 2.94 (m, CH); 2.35 und 1.82 (m, $H_2$-2'und $H_2$-3'); 1.07 und 1.05 (2 s, iBu-Me); 0.87 (s, t-Bu-Me); 0.05 (s, Si-Me).

b) 2-[(2-Methylpropionyl)amino]-8-(2,3-dideoxy-$\beta$-D-glyceropentofuranosyl-8H-imidazo[1,2-a]-s-triazin-4-on

Das nach Beispiel 8a isolierte $\beta$-Anomere wird in trockenem THF (5 ml) gelöst und mit $Bu_4NF$ (1M in THF, 5 ml) versetzt. Man rührt 10 min bei RT, dampft zu einem farbloses Öl ein und chromatographiert an Kieselgel 60 H (Säule: 10 x 6 cm, 1 l $CH_2Cl_2$-MeOH 97:3; 1 l $CH_2$-$Cl_2$-MeOH 9:1). Aus der Hauptzone erhält man das Produkt (110 mg, 93 %) als farblosen Schaum. DC (Kieselgel, $CH_2Cl_2$-MeOH 9:1): $R_f$: = 0.3. $^1$H-NMR ([$D_6$]DMSO): 10.32 (s, NH); 7.77 (d, J = 2.7 Hz, H-7); 7.58 (d, J = 2.7 Hz, H-6); 6.14 (dd, J = 6.4 Hz, 3.7 Hz H-1'); 4.99 (t, J = 5.4 Hz, 5'-OH); 4.11 (m, H-4'); 3.58 (m, $H_2$-5'); 2.93 (m, J = 6.8 Hz); 2.38 (m, $H_2$-2'); 2.03 $H_2$-3'); 1.07 und 1.05(2 $CH_3$).

c) 2-[(2-Methylpropionyl)amino]-8-(2,3-dideoxy-$\alpha$-D-glyceropentofuranosyl-8H-imidazo[1,2-a]-s-triazin-4-on

Das nach Beispiel 8 a isolierte $\alpha$-Anomere wird, wie für Beispiel 8 b beschrieben, umgesetzt und aufgearbeitet. DC (Kieselgel, $CH_2Cl_2$-MeOH 9:1) $R_f$: = 0.3. $^1$H-NMR ([$D_6$]DMSO): 10.34 (s, NH); 7.67 (d, J = 2.6 Hz, H-7); 7.59 (d, J = 2.6 Hz, H-6); 6.21 (dd, J = 6.5 Hz, 3.8 Hz H-1'); 4.83 (t, J = 5.4 Hz, 5'-OH); 4.12 (m, H- 4'); 3.43 (m, $H_2$-5'); 2.90 (m, J = 6.9 Hz, CH); um 2.3 (m, $H_2$-2'); 1.85 und 1.58 $H_2$-3'); 1.07 und 1.05(2 $CH_3$).

d) 8-(2,3-Dideoxy-$\beta$-D-glyceropentofuranosyl-8H-imidazo[1,2-a]-s-triazin-4-on

Das nach Beispiel 8 b isolierte $\beta$-Anomere (75 mg, 0.23 mmol) wird in methanolischem Ammoniak ( 5 ml) gelöst und 2 h bei RT gerührt. Man dampft ein und chromatographiert den öligen Rückstand an Kieselgel 60 H (Säule 6 x 6 cm, LM: $CH_2Cl_2$-MeOH 9:1). Nach Eindampfen der Hauptzone erhält man 40 mg (70 %) Produkt als farblose Kristalle vom Schm. 157-158°C (MeOH). DC (Kieselgel, $CH_2Cl_2$-MeOH 9:1) $R_f$ = 0.2. $^1$H-NMR ([$D_6$]DMSO): 7.50 (d, J = 2.7 Hz, H-7); 7.34 (d, J = 2.7 Hz, H-6); 6.03 (dd, J = 5.8 Hz, 3.4 Hz H-1'); 4.99 (t, J = 5.8 Hz, 5'-OH); 4.06 (m, H- 4'); 3.57 (m, $H_2$-5'); um 2.3 (m, $H_2$-2'); 1.97 (m, $H_2$-3').

e) 8-(2,3-Dideoxy-$\alpha$-D-glyceropentofuranosyl-8H-imidazo[1,2-a]-s-triazin-4-on

Das nach Beispiel 8 c isolierte $\alpha$-Anomere wird, wie für Beispiel 8 d beschrieben, umgesetzt und aufgearbeitet. Der ölige Rückstand wird an Kieselgel 60 (Säule 15 x 6 cm, LM: 1 l $CH_2Cl_2$-MeOH 95:5; 2 l $CH_2Cl_2$-MeOH 9:1) chromatographiert. Nach Eindampfen der Hauptzone erhält man 60 mg (74 %) Produkt als farblosen Schaum. DC (Kieselgel, $CH_2Cl_2$-MeOH 9:1): $R_f$ = 0.2. $^1$H-NMR ([$D_6$]DMSO): 7.39 (d, J = 2.7 Hz, H-7); 7.35 (d, J = 2.7 Hz, H-6); 6.92 (s, br., $NH_2$); 6.10 (dd, J = 3.6 Hz, 6.3 Hz H-1'); 4.81 (t, J = 5.7 Hz, 5'-OH); 4.33 (m, H- 4'); 3.40 (m, $H_2$-5'); 2.40 und 2.20 (2m, $H_2$-2'); 2.20 und 1.83 (2m, $H_2$-3').

Beispiel 9

7-Methoxy-1,(2),(3)-(2',3'-didesoxy-$\alpha$,($\beta$)-D-erythropentofuranosyl)-3H-1,2,3-triazolo[4,5-d]pyrimidin

a) Zu einer Suspension von gepulvertem KOH (750 mg, 14.3 mmol) in wasserfreiem MeCN (50 ml) werden in Abständen von je 10 min TDA-1 (40 ml, 0.12 mmol und 7-Methoxy-3H-1,2,3-triazolo[4,5-d]-pyrimidin (900 mg, 6 mmol) gegeben. Nach weiteren 10 min wird innerhalb von 30 min in Portionen von je 5 ml eine kalte Lösung des nach dem Vergleichsbeispiel unter a) hergestellten t-Butyldimethylsilyl-2',3'-didesoxy-D-ribofuranosylchlorid (12 mmol) in 30 ml THF zugegeben. Die Reaktionsmischung wird für weitere 30 min gerührt, unlösliches Material filtriert und das Filtrat bei 40°C im Ölpumpenvakuum zur Trockene eingedampft. Der sirupöse Rückstand wird sofort flashchromatographiert (Kieselgel 60, Säule: 30 x 3 cm, Petrolether:Essigester, 6:4). Man erhält 3 Hauptfraktionen (I, II, III). Fraktion I enthält 4 Verbindungen, die durch mehrfache Chromatographie (Kieselgel 60, Säule: 25 x 3 cm, Dichlormethan:Aceton, 95:5 und (Säule: 30 x 3 cm; Petrolether:Essigester, 7:3) separiert werden. Fraktion II und III enthalten jeweils eine Verbindung. Die Zuordnung der Regio- und Stereoisomeren erfolgte durch Vergleich der $^{13}$C-NMR- Daten mit denen der entsprechenden Desoxyverbindungen (F. Seela et al. Heterocyclus, 1989, 29, 2193).

b) 7-Methoxy-3-(5'-t-butyldimethylsilyl-2',3'-didesoxy-$\alpha$-D-erythropentofuranosyl)-3H-1,2,3-triazolo[4,5-d]-pyrimidin

Die schnellste Zone von Fraktion I (siehe 9 a) liefert 202 mg (9.2 %) Produkt als farbloses Öl. DC (Kieselgel, Petrolether:Essigester, 7:3) $R_f$ = 0.6. $^1$H-NMR ([$D_6$]DMSO): 0.033 und 0.049 (2 s, 2 $SiCH_3$); 0.86 (s, $SiC(CH_3)_3$); 1.97 (m, 3'-H); 2.66 (m, 2'-H); 3.66 (m, 5'-H); 4.21 (s, $OCH_3$); 4.40 (m, 4'-H); 6.72 (dd, J = 6.8 Hz und 3.3 Hz, 1'-H); 8.76 (s, 5-H).

c) 7-Methoxy-3-(5'-t-butyldimethylsilyl-2',3'-didesoxy-$\beta$-D-erythropentofuranosyl)-3H-1,2,3-triazolo[4,5-d]-pyrimidin

Aus der mittleren Zone von Fraktion I (siehe 9 a) erhält man 195 mg (8.9 %) Produkt als farbloses Öl.

DC (Kieselgel, Dichlormethan:Aceton, 95:5) $R_f$ = 0.65. $^1$H- NMR ([$D_6$]DMSO):-0.194 und -0.15 (2 s, 2 $SiCH_3$); 0.72 (s, $SiC(CH_3)_3$); 2.21 (m, 3'-H); 2.59 und 2.81 (m, 2'-H); 3.55 (dd, J = 11.0 Hz und 5.9 Hz, 5'-H); 4.21 (s, $OCH_3$); 4.26 (m, 4'-H); 6.67 (dd, J = 7.1 Hz und 1.7 Hz, 1'-H); 8.79 (s, 5-H).

d) 7-Methoxy-2-(5'-t-butyldimethylsilyl-2',3'-didesoxy-$\alpha$-D-erythropentofuranosyl)-3H-1,2,3-triazolo[4,5-d]-pyrimidin

Die langsamste Zone von Fraktion I (siehe 9 a) (bezogen auf Laufmittel Dichlormethan: Aceton, 95:5) trennt sich nach erneuter Chromatographie (Laufmittel Petrolether:Essigester, 7:3) in zwei Unterzonen auf. Die schnellere Unterzone ergibt 172 mg (7.8 %) Produkt als farbloses Öl. DC (Kieselgel, Petrolether:Essigester, 7:3) $R_f$ = 0.5. $^1$H-NMR ([$D_6$]DMSO):-0.058 und 0.065 (2 s, 2 $SiCH_3$); 0.86 (s, $SiC(CH_3)_3$); 1.96 (m, 3'-H); 2.5 (m, 2'-H); 3.68 (m, 5'-H); 4.18 (s, $OCH_3$); 4.50 (m, 4'-H); 6.67 (dd, J = 5.9 Hz und 2.7 Hz, 1'-H); 8.76 (s, 5-H).

e) 7-Methoxy-2-(5'-t-butyldimethylsilyl-2',3'didesoxy-$\beta$-D-erythropentofuranosyl)-3H-1,2,3-triazolo[4,5-d]-pyrimidin

Aus der langsameren Unterzone (siehe 9 d) erhält man 168 mg (7.7 %) Produkt als farbloses Öl. DC (Kieselgel, Dichlormethan: Aceton, 95:5) $R_f$ = 0.55.

$^1$H-NMR ([$D_6$]DMSO): -0.163 und 0.111 (2 s, 2 $SiCH_3$); 0.72 (s, $SiC(CH_3)_3$); 2.17 (m, 3'-H); 2.65 und 2.56 (m, 2'-H); 3.66 (m, 5'-H); 4.31 (m, 1'-H); 6.59 (d, J = 5.6 Hz, 1'-H); 8.75 (s, 5-H).

f) 7-Methoxy-1-(5'-t-butyldimethylsilyl-2',3'-didesoxy-$\alpha$-D-erythropentofuranosyl)-3H-1,2,3-triazolo[4,5-d]-pyrimidin

Fraktion II (siehe 9a, Laufmittel Petrolether: Essigester, 6:4) liefert 118 mg (5.4 %) Produkt als farbloses Öl.

DC (Kieselgel, Petrolether:Essigester, 6:4) $R_f$ = 0.5. $^1$H- NMR ([$D_6$]DMSO): 0.048 und 0.063 (2 s, 2 $SiCH_3$); 0.87 (s, $SiC(CH_3)_3$); 1.98 (m, 3'-H); 2.75 und 2.60 (m, 2'-H); 3.66 (m, 5'-H); 4.20 (s, $OCH_3$); 4.33 (m, 4'-H); 6.77 (dd, J = 6.9 Hz und 2.9 Hz, 1'-H); 8.8 (s, 5-H).

g) 7-Methoxy-1-(5'-t-butyldimethylsilyl-2',3'-didesoxy-$\beta$-D-erythropentofuranosyl)-3H-1,2,3-triazolo[4,5-d]-pyrimidin

Fraktion III (siehe 9a, Laufmittel Petrolether: Essigester, 6:4) liefert 175 mg (8.0 %) Produkt als farbloses Öl.

DC (Kieselgel, Petrolether:Essigester, 6:4) $R_f$ = 0.35. $^1$H-NMR ([$D_6$]DMSO): -0.24 und -0.20 (2 s, 2 $SiCH_3$); 0.67 (s, $SiC(CH_3)_3$); 2.15 (m, 3'-H); 2.87 und 2.60 (m, 2'-H); 3.40 (dd, J = 11.1 Hz und 3.9 Hz, 5'-H); 4.28 (m, 4'-H), 6.68 (d, J = 6.9 Hz, 1'-H); 8.75 (s, 5-H).

h) 7-Methoxy-3-(2',3'-didesoxy-$\alpha$-D-erythropentofuranosyl)-3H-1,2,3-triazolo[4,5-d]pyrimidin

500 mg (2.0 mmol) der nach 9 b hergestellten Verbindung werden in 20 ml THF gelöst, mit 2 ml einer 1.1 N Lösung von $Bu_4NF$ in THF versetzt und 2 h bei RT gerührt. Das Lösungsmittel wird abgedampft und der sirupöse Rückstand an Kieselgel 60 (Säule 20 x 3 cm, Dichlormethan:Methanol, 9:1) chromatographiert. Aus der Hauptfraktion erhält man 213 mg (62 %) farbloses, amorphes Produkt. DC (Kieselgel, Dichlormethan:Methanol, 9:1) $R_f$ = 0.55. $^1$H-NMR ([$D_6$]DMSO): 1.97 (m, 3'-H); 2.63 (m, 2'-H); 3.47 (m, 5'-H); 4.21 (s, $OCH_3$); 4.36 (m, 4'-H), 4.81 (t, J = 5.7 Hz, 5'-OH); 6.74 (dd, J = 7.0 Hz und 3.4 Hz, 1'-H); 8.75 (s 5-H).

i) 7-Methoxy-3-(2',3'-didesoxy-$\beta$-D-erythropentofuranosyl)-3H-1,2,3-triazolo[4,5-d]pyrimidin

Die Entschützung der nach 9 c hergestellten Verbindung wird, wie für Beispiel 9 h beschrieben, durchgeführt. Nach 1 h wird zur Trockene eingedampft und an Kieselgel (Säule 20 x 3 cm, Dichlormethan:Methanol, 95:5) chromatographiert. Aus der Hauptfraktion erhält man 291 mg (85 %) Produkt in farblosen Kristallen.

DC (Kieselgel, Dichlormethan:Methanol, 9:1) $R_f$ = 0.65. $^1$H-NMR ([$D_6$]DMSO): 2.24 (m, 3'-H); 2.63 und

2.74 (m, 2'-H); 3.42 (m, 5'-H); 4.22 (m, 4'-H und $OCH_3$); 4.70, (t, J = 5.7 Hz, 5'-OH); 6.66 (dd, J = 7.2 Hz und 2.2 Hz, 1'-H); 8.80 (s, 5-H).

j) 7-Methoxy-2-(2',3'-didesoxy-α-D-erythropentofuranosyl)-3H-1,2,3-triazolo[4,5-d]pyrimidin

500 mg (2.0 mmol) der nach 9 d hergestellten Verbindung wird, wie in Beispiel 9 h beschrieben, behandelt. Nach einer Reaktionszeit von 1.25 h wird das Lösungsmittel abgedamptt und der ölige Rückstand an Kieselgel 60 (Säule 20 x 3 cm, Dichlormethan:Methanol, 95:5) chromatographiert. Eindampfen der Hauptzone ergibt das Produkt als kristalline Verbindung. DC (Kieselgel, Dichlormethan:Methanol, 9:1) $R_f$ = 0.45. $^1$H-NMR ([$D_6$]DMSO): 1.94 und 2.34 (2m, 3'-H); 2.56 (m, 2'-H); 3.48 (m, 5'-H); 4.18 (s, $OCH_3$); 4.46 (m, 4'-H); 4.85, (t, J = 5.8 Hz, 5'-OH); 6.67 (dd, J = 6.2 Hz und 2.5 Hz, 1'-H); 8.76 (s, 5-H).

k) 7-Methoxy-2-(2',3'-didesoxy-β-D-erythropentofuranosyl)-3H-1,2,3,-triazolo[4,5-d]pyrimidin

500 mg (2.0 mmol) der nach 9 e hergestellten Verbindung wird, wie in Beispiel 9 h beschrieben, behandelt. Nach einer Reaktionszeit von 1 h wir zur Trockene eingedampft und an Kieselgel 60 (Säule: 20 x 3 cm, Dichlormethan:Methanol, 9:1) chromatographiert. Man erhält so 286 mg (84 %) amorphes Produkt.

DC (Kieselgel, Dichlormethan:Methanol, 9:1) $R_f$ = 0.7. $^1$H- NMR ([$D_6$]DMSO): 2.17 (2m, 3'-H); 2.57 (m, 2'-H); 3.50 (m, 5'-H); 4.16 (s, $OCH_3$); 4.28 (m, 4'-H); 4.76, (t, J = 5.6 Hz, 5'-OH); 6.60 (dd, J = 4.8 Hz und 3.2 Hz, 1'-H); 8.75 (s, 5-H).

l) 7-Methoxy-1-(2',3'-didesoxy-α-D-erythropentofuranosyl)-3H-1,2,3,-triazolo[4,5-d]pyrimidin

500 mg (2.0 mmol) der nach 9 f hergestellten Verbindung wird, wie in Beispiel 9 h beschrieben, behandelt. Nach einer Reaktionszeit von 1 h wird zur Trockene eingedampft und an Kieselgel 60 (Säule: 20 x 3 cm, Dichlormethan:Methanol, 9:1) chromatographiert. Man erhält aus der Hauptfraktion 246 mg (72 %) Produkt.

DC (Kieselgel, Dichlormethan:Methanol, 9:1) $R_f$ = 0.6. $^1$H- NMR ([$D_6$]DMSO): 1.97 (m, 3'-H); 2.71 und 2.60 (m, 2'-H); 3.47 (m, 5'-H); 4.21 (s, $OCH_3$); 4.29 (m, 4'-H); 4.81, (t, J = 5.7 Hz, 5'-OH); 6.79 (dd, J = 7.4 Hz und 3.3 Hz, 1'-H); 8.75 (s, 5-H).

m) 7-Methoxy-1-(2',3'-didesoxy-β-D-erythropentofuranosyl)-3H-1,2,3,-triazolo[4,5-d]pyrimidin

500 mg (2.0 mmol) der nach 9 g hergestellten Verbindung wird, wie in Beispiel 9 h beschrieben, behandelt. Nach einer Reaktionszeit von 0.5 h wird zur Trockene eingedampft und an Kieselgel 60 (Säule: 20 x 3 cm, Dichlormethan:Methanol, 9:1) chromatographiert. Man erhält so 303 mg (89 %) Produkt.

DC (Kieselgel, Dichlormethan:Methanol, 9:1) $R_f$ = 0.5. $^1$H- NMR ([$D_6$]DMSO): 2.19 (m, 3'-H); 2.60 und 2.79 (2m, 2'-H); 3.33 (m, 5'-H); 4.20 (s, $OCH_3$); 4.23 (m, 4'-H); 4.64, (t, J = 5.6 Hz, 5'-OH); 6.69 (d, J = 5.7 Hz, 1'-H); 8.75 (s, 5-H).

## Beispiel 10

7-Amino-3-(2',3'-didesoxy-α-D-erythropentofuranosyl)-3H-1,2,3-triazolo [4,5-d]pyrimidin

100 mg (0.4 mmol) der nach 9 h hergestellten Verbindung werden in 10 ml methanolischem $NH_3$ - (gesättigt bei 0°C) bei RT 24 h gerührt. Nachdem das Lösungsmittel abgedampft ist, wird das Rohprodukt durch Chromatographie (Kieselgel 60, Säule: 10 x 2 cm, Dichlormethan:Methanol, 9:1) gereinigt. Aus der Hauptfraktion erhält man 69 mg (74 %) Produkt als kristallinen Feststoff.

DC (Kieselgel, Dichlormethan:Methanol, 9:1) $R_f$ = 0.3. $^1$H-NMR ([$D_6$]DMSO): 1.91 und 2.36 (2m, 3'-H); 2.7 - 2.5 (2'-H); 3.46 (m, 5'-H); 4.33 (m, 4'-H); 4.80, (t, J = 5.7 Hz, 5'-OH); 6.61 (dd, J = 7.0 und 3.5 Hz, 1'-H); 8.12 und 8.45 (2s, 2 NH); 8.32 (s, 5-H).

## Beispiel 11

7-Amino-3-(2',3'-didesoxy-β-D-erythropentofuranosyl)-3H-1,2,3-triazolo [4,5-d]pyrimidin

100 mg (0.4 mmol) der nach 9 i hergestellten Verbindung werden, wie in Beispiel 10 beschrieben, behandelt. Nachdem das Lösungsmittel abgedampft ist, wird das Rohprodukt durch Chromatographie (Kieselgel 60, Säule: 10 x 2 cm, Dichlormethan:Methanol, 9:1) gereinigt. Nach Eindampfen der Hauptfraktion erhält man 83 mg (88 %) Produkt, das aus Methanol in farblosen Nadeln vom Schmp. 182°C kristallisiert.

DC (Kieselgel, Dichlormethan:Methanol, 9:1) $R_f$ = 0.54. $^1$H-NMR ([$D_6$]DMSO): 2.24 (m, 3'-H); 2.59 und 2.70 (2m, 2'-H); 3.46 (m, 5'-H); 4.81 (t, J = 5.7 Hz, 5'-OH); 6.55 (dd, J = 7.1 und 2.7 Hz, 1'-H); 8.47 und 8.14 (2s, 2 NH); 8.33 (s, 5-H).

Beispiel 12

7-Amino-2-(2',3'-didesoxy-α-D-erythropentofuranosyl)-3H-1,2,3-triazolo [4,5-d]pyrimidin

100 mg der nach 9 j hergestellten Verbindung werden, wie in Beispiel 10 beschrieben, behandelt. Die Reaktion erfordert 7h. Nach Abdampfen des Lösungsmittels und Chromatographie an Kieselgel (Säule 10 x 2 cm, Dichlormethan:Methanol, 9:1) erhält man kristallines Produkt.
DC (Kieselgel, Dichlormethan:Methanol, 9:1) $R_f$ = 0.15. $^1$H-NMR ([$D_6$]DMSO): 1.94 und 2.35 (2m, 3'-H); 2.55 (m, 2'-H); 3.49 (m, 5'-H); 4.43 (m, 4'-H); 4.85, (t, J = 5.3 Hz, 5'-OH); 6.56 (pt, J = 4.5 Hz, 1'-H); 8.1 (s, $NH_2$); 8.32 (s, 5-H).

Beispiel 13

7-Amino-2-(2',3'-didesoxy-β-D-erythropentofuranosyl)-3H-1,2,3-triazolo [4,5-d]pyrimidin

100 mg der nach 9 k hergestellten Verbindung werden, wie in Beispiel 10 beschrieben, behandelt. Nach 8 h wird zur Trockene eingedampft und an Kieselgel (Säule 10 x 2 cm, Dichlormethan:Methanol, 9:1) chromatographiert.
DC (Kieselgel, Dichlormethan:Methanol, 9:1) $R_f$ = 0.35. $^1$H-NMR ([$D_6$]DMSO): 2.17 (m, 3'-H); 2.54 (m, 2'-H); 3.48 (pt, J = 5.6 Hz, 5'-H); 4.24 (m, 4'-H); 4.75, (t, J = 5.6 Hz, 5'-OH); 6.47 (d, J = 4.4 Hz, 1'-H); 8.10 (s, NH); 8.30 (s, NH und 5-H).

Beispiel 14

7-Amino-1-(2',3'-didesoxy-α-D-erythropentofuranosyl)-3H-1,2,3-triazolo [4,5-d]pyrimidin

100 mg der nach 9 l hergestellten Verbindung werden, wie in Beispiel 10 beschrieben, behandelt. Nach 36h wird das Lösungsmittel abgedampft und das Rohprodukt durch Chromatographie (Kieselgel 60, Säule: 10 x 2 cm, Dichlormethan:Methanol, 9:1) gereinigt. Nach Eindampfen der Hauptfraktion erhält man 63 mg (67 %) Produkt, das aus Aceton in feinen Nadeln kristallisiert.
DC (Kieselgel, Dichlormethan:Methanol, 9:1) $R_f$ = 0.25. $^1$H-NMR ([$D_6$]DMSO): 1.93 und 2.15 (2m, 3'-H); 3.51 (m, 5'-H); 4.17 (m, 4'-H); 4.91, (t, J = 5.7 Hz, 5'-OH); 6.76 (dd, J = 6.3 Hz und 1.7 Hz, 1'-H); 7.78 (s, $NH_2$); 8.35 (s, 5-H).

Beispiel 15

7-Amino-1-(2',3'-didesoxy-β-D-erythropentofuranosyl)-3H-1,2,3-triazolo [4,5-d]pyrimidin

100 mg der nach 9 m hergestellten Verbindung werden, wie in Beispiel 10 beschrieben, behandelt. Nach 36 h wird zur Trockene eingedampft und an Kieselgel (Säule: 10 x 2 cm, Dichlormethan:Methanol, 9:1) chromatographiert. Aus der Hauptzone ergeben sich 58 mg (62 %) kristallines Produkt.
DC (Kieselgel, Dichlormethan:Methanol, 9:1) $R_f$ = 0.25. $^1$H-NMR ([$D_6$]DMSO): 1.93 und 2.16 (2m, 3'-H); 2.49 (m, 2'-H); 3.15 (m, 5'-H); 4.36 (m, 4'-H); 4.77 (t, J = 5.2 Hz, 5'-OH); 6.64 (dd, J = 4.5 Hz, 1'-H); 7.76 (s, $NH_2$); 8.33 (s, 5-H).

Beispiel 16

4-Amino-1-(2,3-didesoxy-α-(β)-D-glyceropentofuranosyl)-1H-imidazo [4,5-c]pyridin

a) 4-Chloro-1-{5-0-[(1,1-dimethylethyl)dimethylsilyl]-2,3-dideoxy-α,(β)-D-glyceropentofuranosyl}-1H-imidazo[4,5-c]pyridin 4-Chlorimidazo[4,5-c]pyridin (809 mg, 5.3 mmol) wird, wie in Beispiel 8 a beschrieben, mit 5-0-[(1,1-Dimethylethyl)dimethylsilyl]-2,3-dideoxy-D-glyceropentofuranosylchlorid (bereitet aus 2.6 g, 11 mmol, des Lactols) umgesetzt und aufgearbeitet. [DC (Kieselgel, $CH_2Cl_2$-MeOH, 95:5): $R_f$ = 0.77]. Das als farbloses Öl anfallende Anomerengemisch wird in EtOAc-Petrolether 3:7 gelöst und an Kieselgel 60H (Säule 35 x 6 cm, 0.8 bar, EtOAc-Petrolether 3:7) chromatographiert. Aus der schneller wandernden Zone erhält man nach Eindampfen 390 mg (23 %) des a-Anomeren als farblose Kristalle vom Schmp. 65-68 °C (EtOAc).
$^1$H-NMR ([$D_6$]DMSO): 8.63 (s, H-1); 8.16 (d, J = 5.6 Hz, H-6); 7.73 (d, J = 5.6 Hz, H-7); 6.39 (dd, J =

6.4, 4.0 Hz, H-1'); 4.39 (m, H-4'); 3.65 (m, $H_2$-5'); um 2.4 (m, $H_2$-2'); 2.20 (m, $H_a$-3'); 1.94 (m, $H_\beta$-3'); 0.87 (s, t-Bu-Me); 0.06 (s, Si-Me). Aus der langsamer wandernden Zone erhält man nach Eindampfen 420 mg (25 %) des $\beta$-Anomeren als farblosen Schaum.

$^1$H-NMR ([$D_6$]DMSO): 8.65 (s, H-2); 8.14 (d, J = 5.6 Hz, H-6); 7.75 (d, J = 5.6 Hz, H-7); 6.31 (pt, J = 4.3 Hz, H-1'); 4.20 (m, H-4'); 3.69 (m, $H_2$-5'); um 2.5 (m, $H_2$-2'); 2.04 (m, $H_2$-3'); 0.78 (s, t-Bu-Me); 0.05 - 0.07 (s, Si-Me).

b) 4-Chloro-1-(2,3-dideoxy-$\beta$-D-glyceropentofuranosyl)-1H-imidazo [4,5c]pyridin

Das in Beispiel 16 a erhaltene $\beta$-Anomere (300 mg, 0.9 mmol) wird in THF (5 ml) gelöst und mit $Bu_4NF$ (1 M in THF, 5 ml) versetzt und 15 min bei RT gerührt. Man dampft zu einem farblosen Öl ein und chromatographiert an Kieselgel 60H (Säule 10 x 6 cm, 0.5 bar, $CH_2Cl_2$-MeOH, 9:1). Nach Eindampfen der Hauptzone erhält man 170 mg (86 %) Produkt als farbloses Öl.

DC (Kieselgel, $CH_2Cl_2$-MeOH 9:1) $R_f$ = 0.45.

$^1$H-NMR ([$D_6$]DMSO): 8.73 (s, H-2); 8.15 (d, J = 5.6 Hz, H-6); 7.78 (d, J = 5.6 Hz, H-7); 6.32 (dd, J = 6.4 Hz, 3.4 Hz, H-1'); 4.98 (t, J = 5.3 Hz, 5'-OH); 4.13 (m, H-4'); 3.53 (m, $H_2$-5'); um 2.4 (m, $H_2$-2'); 2.05 (m, $H_2$-3').

c) 4-Chloro-1-(2,3-dideoxy-$\alpha$-D-glyceropentofuranosyl)-1H-imidazo [4,5-c]pyridin

Das in Beispiel 16 a erhaltene a-Anomere (300 mg, 0.9 mmol) wird wie in Beispiel 12b beschrieben, umgesetzt und aufgearbeitet. Man erhält 160 mg (81 %) farblose Kristalle.

DC (Kieselgel, $CH_2Cl_2$-MeOH 9:1) $R_f$ = 0.42.

$^1$H-NMR ([$D_6$]DMSO): 8.64 (s, H-2); 8.17 (d, J = 5.6 Hz, H-6); 7.74 (d, J = 5.6 Hz, H-7); 6.39 (dd, J = 6.2 Hz, 4.1 Hz, H-1'); 4.86 (t, J = 5.3 Hz, 5'-OH); 4.34 (m, H-4'); 3.46 (m, $H_2$-5'); um 2.5 (m, $H_2$-2'); 2.19 und 1.93 (2m, $H_2$- 3').

d) 4-Amino-1-(2,3-dideoxy-$\beta$-D-glyceropentofuranosyl)-1H-imidazo[4,5-c]pyridin

Das in Beispiel 16 b erhaltene Produkt (100 mg, 0.46 mmol) wird mit Hydrazinhydrat (100 %, 10 ml) versetzt und 90 min unter $N_2$ bei 90-100°C gerührt. Nach Abdampfen überschüssigen Hydrazinhydrats wird mehrfach mit EtOH nachgedampft und der Rückstand in EtOH (25 ml) gelöst. Man fügt 2 g Raney Nickel hinzu und kocht 2 h unter Rückfluß. Nach Abfiltrieren des Katalysators wird das Filtrat eingedampft und der Rückstand an Kieselgel 60H (Säule 6 x 6 cm, 0.5 bar, $CH_2Cl_2$-MeOH, 9:1) chromatographiert. Nach Eindampfen der Hauptzone erhält man 40 mg (37 %) der Titelverbindung als farblosen Schaum.

DC (Kieselgel, $CH_2Cl_2$-MeOH 9:1) $R_f$ = 0.4.

$^1$H-NMR ([$D_6$]DMSO): 8.33 (s, H-2); 7.67 (d, J = 5.8 Hz, H-6); 6.65 (d, J = 5.8 Hz, H-7); 6.25 (s, br., $NH_2$); 6.14 (dd, J = 6.5 Hz, 3.9 Hz, H-1'); 4.12 (m, H-4'); 3.53 (m, $H_2$-5'); 2.33 (m, $H_2$-2'); 2.01 (2m, $H_2$-3').

## Beispiel 17

### 1-(2,3-Didesoxy-$\beta$-D-glyceropentofuranosyl)imidazo[1,2-a]pyrimidin- 2-on

a) 7-Chlor-1-(2,3-didesoxy-5-0-(tert-butyldimethylsilyl)-$\beta$-D-glyceropentofuranosyl)imidazo[1,2-a]pyrimidin-5-on und sein a-Anomer

1.0 g (5.9 mmol) 7-Chlor-imidazo[1,2-a]pyrimidin-5-on (R.G. Revankar et al. Anm. N.Y. Acad. Sci. 255, 166, 1975) werden in einem Gemisch aus 10 ml DMF und 50 ml THF gelöst und mit 3.0 KOH und 30 ml TDA-1 30 min bei Raumtemperatur gerührt. In 4 Portionen wird das nach dem Vergleichsbeispiel unter a) in situ hergestellte 5-O-[(1,1 - Dimethylethyl)dimethylsilyl]-2,3-didesoxy-D-glycero-pentofuranosyl-chlorid (13 mmol) zugespritzt. Man rührt weitere 45 min bei Raumtemperatur, filtriert, gibt 300 ml gesättigte $NaHCO_3$ Lösung dazu und extrahiert die wässrige Phase mit $CHCl_3$. Der organische Extrakt wird mit $NaSO_4$ getrocknet und im Vakuum eingedampft. Chromathographie an Kieselgel 60 liefert 450 mg (20 %) des $\alpha$-Anomeren (schnelle Zone) als farbloses Öl und 590 mg (26 %) des $\beta$-Anomeren (langsame Zone) als farblose Kristalle vom Schmp. 63°C (Ether).

($\beta$-Anomer): $^1$H NMR ([$D_6$]DMSO): d = 0.06 (s, 6H, $CH_3$), 0.87 (s, 9H, $CH_3$), 1.68 (m, 2H, H-3'), 2.25 (m, 2H, H-2'), 3.62 (m, 2H, H-5'), 4.45 (m, 1H, H-4'), 5.99 (s, 1H, H-6), 6.27 (dd J= 3.9 Hz, J= 6.8 Hz, 1H, H-1'), 7.73 (d, J= 2.7 Hz, 1H, H-3), 7.80 (d, J= 2.7 Hz, 1H, H-2);

($\alpha$-Anomer): d = 0.02 (s, 6H, $CH_3$), 0.64 (s, 9H, $CH_3$), 2.02 (m, 2H, H-3'), 2.38 (m, 2H, H-2'), 3.76 (m, 2H, H-5'), 4.14 (m, 1H, H-4'), 5.99 (s, 1H, H-6), 6.21 (m, 1H, H-1'), 7.72 (d, J= 2.4 Hz, 1H, H-3), 7.79 (d, J=2.4 Hz, 1H, H-2).

b) 7-Chlor-1-(2,3-didesoxy-$\beta$-D-glycero-pentofuranosyl)imidazo [1,2-a]pyrimidin-5-on

250 mg (0.65 mmol) des nach a) hergestellten $\beta$-Anomeren wird in THF gelöst, mit 2 ml $Bu_4NF$ (1M Lsg.

in THF) versetzt und bei Raumtemperatur 60 min gerührt. Nach dem Abdampfen des Lösungsmittels chromathographiert man an einer 20 x 2 cm Kieselgel 60 Säule. Die Hauptzone liefert das Produkt als farblose Kristalle (74 %) vom Schmp. 157°C (Propanol-2).

$^1$H NMR ([$D_6$]DMSO): d = 2.00 (m, 2H, H-3'), 2.37 (m, 2H, H-2'), 3.60 (m, 2H, H-5'), 4.10 (m, 1H, H-4'), 5.01 (t, J = 5.4 Hz, 1H, OH), 5.99 (s, 1H, H-6), 6.22 (dd J = 2.7 Hz, J = 6.7 Hz, 1H, H-1'), 7.73 (d, J = 2.6 Hz, 1H, H-3), 7.92 (d, J = 2.6 Hz, 1H, H-2).

c) 1-(2,3-Didesoxy-β-D-glycero-pentofuranosyl)imidazo[1,2-a]pyrimidin-5-on

60 mg (0.22 mmol) der nach b) hergestellten Verbindung werden in 50 ml Ethanol gelöst. Man gibt 4 ml konz. $NH_3$ dazu und hydriert mit 100 mg Pd (10 % Pd auf C) 14 H bei RT. Der Katalysator wird abfiltriert, mit heißem Ethanol gewaschen und das Filtrat eingedampft. Flash-Chromatographie an Kieselgel 60 liefert 12 mg (23 %) Produkt.

$^1$H NMR ([$D_6$]DMSO) d = 2.00 (m, 2H, H-3'), 2.30 (m, 2H, H-2'), 3.60 (m, 2H, H-5'), 4.10 (m, 1H, H-4'), 5.03 (t, J = 5.4 Hz, 1H, OH), 5.90 (d, J = 6.3 Hz, 1H, H-6), 6.28 (dd, J = 3.2 Hz, J = 6.8 Hz, 1H, H-1'), 7.68 (d, J = 2.7 Hz, 1H, H-3), 7.86 (d, J = 2.7 Hz, 1H, H-2), 7.94 (d, J = 6.3 Hz, 1H, H-7).

Beispiel 18

7-Amino-1-(2,3-didesoxy-β-D-glycero-pentofuranosyl)imidazo [1,2-a]pyrimidin-5-on

Die Titelverbindung erhält man aus 60 mg (0.22 mmol) der nach Beispiel 17 b) hergestellten Verbindung in Analogie zu der Vorschrift für die entsprechende Riboverbindung (R.G. Revankar et al. Anm. N.Y. Sci. 255, 166, 1975). DC (Kieselgel, Dichlormethan: Methanol, 9:1) $R_f = 0.35$.

**Patentansprüche**

1. Nucleosid-Derivate der allgemeinen Formel I

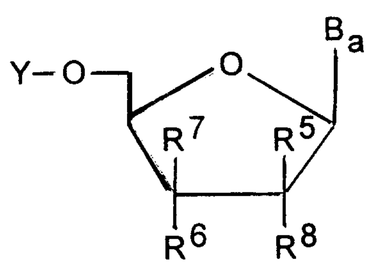

(I)

in der

$B_a$ eine Benzimidazolyl- (B), Pyrrolopyridinyl- (C), Imidazopyridinyl- (D), Triazolopyrimidinyl- (E), Imidazotriazinyl-(F) oder Imidazopyrimidinylgruppe (G)

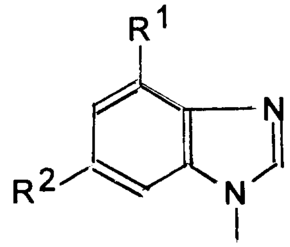

(B)

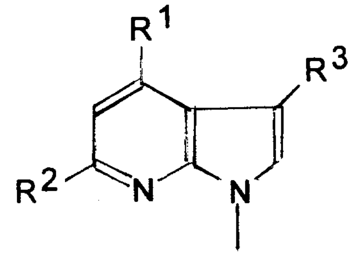

(C)

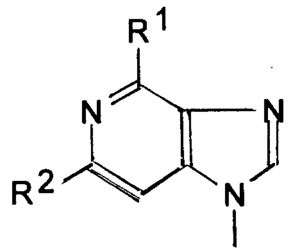

(D)

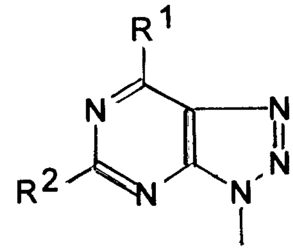

(E)

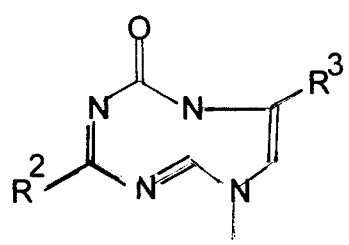

(F)

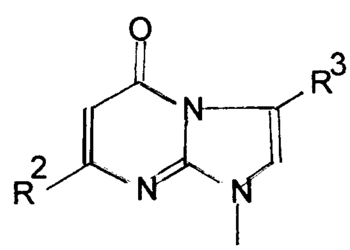

(G)

bedeutet, wobei

$R^1$, $R^2$, $R^3$, die gleich oder verschieden sein können, Wasserstoff, Halogen, eine $C_1$-$C_7$-Alkyl-, $C_2$-$C_7$-Alkenyl-, Hydroxy-, Mercapto-, $C_1$-$C_7$-Alkylthio-, $C_1$-$C_7$-Alkyloxy-, $C_2$-$C_7$-Alkenyloxy-, Ar-$C_1$-$C_5$-alkyl-, Ar-$C_2$-$C_5$-alkenyl-, Ar-$C_1$-$C_5$-alkyloxy-, Ar-$C_2$-$C_5$-alkenyloxy-, Aryloxy-, Nitro-, Amino-$C_1$-$C_7$-alkyl-, $C_1$-$C_7$-Alkylamino-$C_1$-$C_7$-alkyl-, Di-$C_1$-$C_7$-alkylamino-$C_1$-$C_7$-alkyl-, Amino-, eine substituierte Aminogruppe-$NHR^4$, bzw.-$N(R^4)_2$, oder eine Iminogruppe $-N=CH-R^4$ bedeuten,
wobei $R^4$ eine $C_1$-$C_7$-Alkyl-, $C_2$-$C_7$-Alkenyl-, $C_3$-$C_7$-Cycloalkyl-, $C_3$-$C_7$-Cycloalkyl-$C_1$-$C_7$-alkyl-, $C_3$-$C_7$-Cycloalkenyl-, $C_1$-$C_7$-Alkoxy-$C_1$-$C_7$-alkyl-, Halogen-$C_1$-$C_7$-alkyl-, Hydroxy-$C_1$-$C_7$-alkyl-, Ar-$C_1$-$C_5$-alkyl-, Ar-$C_2$-$C_5$-alkenyl-, Hetaryl-$C_1$-$C_5$-alkyl- oder Hetaryl-$C_2$-$C_5$-alkenylgruppe, wobei der Aryl- bzw. Hetarylteil ein-, zwei- oder dreifach durch $C_1$-$C_6$-Alkyl, $C_2$-$C_6$- Alkenyl-, $C_1$-$C_6$-Alkoxy, Halogen oder Hydroxy substituiert sein kann, oder $R^4$ eine Amino-$C_1$-$C_7$-alkyl-, $C_1$-$C_7$-Alkylamino-$C_1$-$C_7$-alkyl- oder Di-$C_1$-$C_7$-alkylamino-$C_1$-$C_7$- alkylgruppe

bedeuten kann und im Fall der $-NHR^4$ und $-N=CH-R^4$-Gruppe, $R^4$ zusätzlich eine Amino-, $C_1$-$C_7$-Alkylamino-, Di-$C_1$-$C_7$-alkylamino- oder $C_1$-$C_7$-Alkoxygruppe sein kann, oder im Fall der $N(R^4)_2$-Gruppe die beiden Stickstoffsubstituenten $R^4$ zusammen eine $C_1$-$C_7$-Alkylidengruppe bilden, die ihrerseits durch eine $C_1$-$C_7$-Alkoxy-, $C_1$-$C_7$-Alkylamino- oder Di-$C_1$-$C_7$-Alkylaminogruppe substituiert sein kann,

$R^5$, $R^6$, $R^7$ und $R^8$ jeweils Wasserstoff bzw. einer oder zwei der Reste $R^5$, $R^6$, $R^7$ oder $R^8$ eine Hydroxy-, Halogen-, Cyano- oder Azidogruppe bedeuten, oder $R^5$ und $R^7$ zusammen eine weitere Bindung zwischen C-2' und C-3' darstellen können, und

Y Wasserstoff oder eine $C_1$-$C_7$-Alkylcarbonyl-, Monophosphat-, Diphosphat-oder Triphosphatgruppe darstellt,

mit der Maßgabe, daß

a) für den Fall, daß $R^6$ eine Hydroxygruppe ist, $R^8$ nicht ein Wasserstoffatom oder eine Hydroxygruppe sein kann,

b) für den Fall, daß $B_a$ die Gruppe (D) und $R^2$ Wasserstoff ist, $R^1$ keine Chlor- oder Aminogruppe und $R^6$ kein Wasserstoffatom sein kann, und

c) für den Fall, daß $B_a$ die Gruppe (E) und $R^1$ die Aminogruppe ist, $R^5$ und $R^7$ gemeinsam keine Bindung darstellen können,

sowie deren mögliche $\alpha$- oder $\beta$-Anomere, $N^7$-, $N^8$- oder $N^9$-Regioisomere (Purin-Nomenklatur), Tautomere und Salze, sowie Nucleinsäuren, die Verbindungen der Formel I als Bausteine enthalten.

**2.** Nucleoside der Formel (I) gemäß Anspruch 1, dadurch gekennzeichnet, daß
$R^1$ Wasserstoff, eine Amino-, $C_1$-$C_6$-Alkoxy-, Halogen-oder Nitrogruppe,
$R^2$ Wasserstoff oder eine Halogen- oder Aminogruppe, und
$R^3$ Wasserstoff bedeuten.

**3.** Nucleoside der Formel (I) gemäß Anspruch 1 oder 2, dadurch gekennzeichnet, daß $B_a$ die Gruppe (B), und $R^1$, $R^2$ und $R^5$-$R^8$ ein Wasserstoffatom bedeuten.

**4.** Nucleoside der Formel (I) gemäß Anspruch 1 oder 2, dadurch gekennzeichnet, daß $B_a$ die Gruppe (C) und $R^1$ ein Wasserstoffatom oder eine Amino- oder Nitrogruppe, und $R^2$, $R^3$, $R^5$-$R^8$ jeweils ein Wasserstoffatom und $R^5$ und $R^7$ zusammen auch eine Bindung bedeuten.

**5.** Nucleoside der Formel (I) gemäß Anspruch 1 oder 2, dadurch gekennzeichnet, daß $B_a$ die Gruppe (D) und $R^1$ eine Aminogruppe oder ein Chloratom und $R^2$, $R^5$-$R^8$ jeweils ein Wasserstoffatom bedeuten.

**6.** Nucleoside der Formel (I) gemäß Anspruch 1 oder 2, dadurch gekennzeichnet, daß $B_a$ die Gruppe (E) und $R^1$ eine Amino- oder $C_1$-$C_6$-Alkoxygruppe und $R^2$ und $R^5$-$R^8$ jeweils ein Wasserstoffatom bedeuten.

**7.** Nucleoside der Formel (I) gemäß Anspruch 1 oder 2, dadurch gekennzeichnet, daß $B_a$ die Gruppe (F) und $R^2$ Wasserstoff oder eine Aminogruppe und $R^3$, $R^5$-$R^8$ ein Wasserstoffatom bedeuten.

**8.** Nucleoside der Formel (I) gemäß Anspruch 1 oder 2, dadurch gekennzeichnet, daß $B_a$ die Gruppe (G) und $R^2$ Wasserstoff oder eine Aminogruppe oder ein Chloratom und $R^3$, $R^5$-$R^8$ ein Wasserstoffatom bedeuten.

**9.** Verfahren zur Herstellung der Verbindungen nach den Ansprüchen 1-8, dadurch gekennzeichnet, daß man Verbindungen der Formel II,

$B_a$-X (II)

in der
$B_a$ eine Benzimidazolyl- (B), Pyrrolopyridinyl- (C), Imidazopyridinyl- (D), Triazolopyrimidinyl- (E), Imidazotriazinyl-(F) oder Imidazopyrimidinyl-gruppe (G) bedeutet und X ein Wasserstoffatom oder eine Alkalimetallgruppe ist,
mit einer Verbindung der Formel III

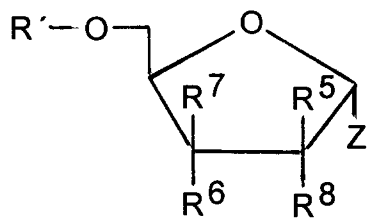

(III)

in der
$R^5$, $R^6$, $R^7$ und $R^8$ jeweils Wasserstoff bedeuten, oder $R^5$ und $R^7$ zusammen eine weitere Bindung zwischen C-2' und C-3' darstellen können, R' eine Sauerstoffschutzgruppe und Z eine reaktive Gruppe bedeuten
zu Verbindungen der Formel IV

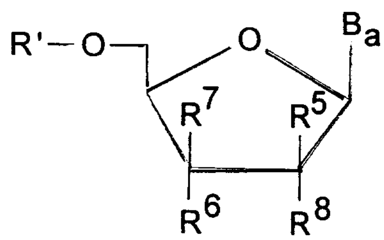

(IV)

in der
$B_a$, $R^1$, $R^2$, $R^3$, $R^5$, $R^6$, $R^7$, $R^8$ und R' die oben angegebene Bedeutung haben,
umsetzt und gegebenenfalls vorhandene Sauerstoffschutzgruppen abspaltet, und gewünschtenfalls anschließend Verbindungen der Formel I, in denen Y Wasserstoff bedeutet, in bekannter Weise in die Mono-, Di- oder Triphosphate überführt, und gewünschtenfalls erhaltene freie Basen bzw. Säuren in die entsprechenden Salze oder erhaltene Salze in die entsprechenden freien Basen bzw. Säuren umwandelt.

**10.** Verwendung von Verbindungen gemäß Anspruch 1-8 bei der DNA-Sequenzierung.

**11.** Verwendung von Verbindungen gemäß Anspruch 1-8 zur Herstellung von Arzneimitteln mit antiviraler Wirkung.

**12.** Arzneimittel, enthaltend mindestens eine Verbindung gemäß Anspruch 1-8 sowie übliche Träger- und Hilfsstoffe.